# EUROPEAN PATENT SPECIFICATION

(11) **EP 2 931 292 B1**
(45) Date of publication and mention of the grant of the patent: **13.06.2018**
(21) Application number: 13821241.0
(22) Date of filing: 13.12.2013
(51) Int. Cl.: A61K 33/06, A61K 33/14, A61K 31/77, A61K 45/00, A61K 45/06, A61P 25/00, A61K 9/00, A61K 9/08, A61K 47/34

(54) **COMPOSITIONS AND METHODS COMPRISING POLYETHYLENE GLYCOL AND MAGNESIUM FOR TREATMENT OF NEURONAL INJURY**
ZUSAMMENSETZUNGEN UND VERFAHREN MIT POLYETHYLENGLYKOL UND MAGNESIUM ZUR BEHANDLUNG VON NEURONALEN LÄSIONEN
DES COMPOSITIONS ET DES PROCÉDÉS COMPRENANT DU POLYÉTHYLÈNEGLYCOL ET DU MAGNÉSIUM POUR LE TRAITEMENT DE LÉSIONS NEURONALES

(30) Priority: 13.12.2012 US 201261736917 P; 23.09.2013 US 201361881174 P; 27.09.2013 US 201361883713 P
(43) Date of publication of application: 21.10.2015
(73) Proprietor: Warsaw Orthopedic, Inc., Warsaw, IN 46582 (US); Josee, Roy, Germantown, TN 38138 (US)
(72) Inventor: JOSEE, Roy, Germantown, TN 38138 (US)
(74) Representative: Huygens, Arthur Victor
(86) International application number: PCT/US2013/075168
(87) International publication number: WO 2014/093918

(56) References cited:
- WO-A2-2010/111215
- WO-A2-2010/111221
- LEE JAE H T ET AL: "Magnesium in a polyethylene glycol formulation provides neuroprotection after unilateral cervical spinal cord injury", SPINE, LIPPINCOTT WILLIAMS & WILKINS, UNITED STATES, vol. 35, no. 23, 1 November 2010 (2010-11-01), pages 2041-2048, XP009175247, ISSN: 1528-1159
- KWON BRIAN K ET AL: "Magnesium chloride in a polyethylene glycol formulation as a neuroprotective therapy for acute spinal cord injury: preclinical refinement and optimization.", JOURNAL OF NEUROTRAUMA AUG 2009, vol. 26, no. 8, August 2009 (2009-08), pages 1379-1393, XP002720052, ISSN: 1557-9042

## Description

This application claims the benefit of the filing date of U.S. Provisional Application No. 61/883,713 filed September 27, 2013, entitled "Compositions and Methods Comprising Polyethylene Glycol and Magnesium for Treatment of Neuronal Injury", and U.S. Provisional Application No. 61/881,174, filed September 23, 2013, entitled "Compositions and Methods Comprising Polyethylene Glycol and Magnesium for Treatment of Neuronal Injury" and U.S. Provisional Application No. 61/736,917, filed December 13, 2012, entitled "Compositions and Methods Comprising Polyethylene Glycol and Magnesium for Treatment of Neuronal Injury".

### BACKGROUND

Neuronal injury can cause sudden, devastating and often lifelong neurologic impairment for the affected individual. One example of neuronal injury is spinal cord injury (SCI). SCI occurs when a traumatic event results in damage to cells within the spinal cord or severs the nerve tracts that relay signals up and down the spinal cord. At times, there is no evidence of penetrating or transection injury. According to the National Spinal Cord Injury Statistical Center (NSCISC), there are between 183,000 and 230,000 people in the United States, and approximately 2 million people worldwide living with a spinal cord injury. Each year, there are 12,000 new injuries reported. Males account for the majority of spinal cord injury patients with 50-70% of those occurring in those aged 15-35. Despite the considerable promise of research in preclinical models and some success in early phase studies, there are no pharmacologic products approved for the treatment of SCI.

SCI is diagnosed via a neurological exam which establishes the level and severity of injury on the American Spinal Injury Association (ASIA) impairment scale (AIS). Several diagnostic techniques employed during the neurological examination include X-rays, computed tomography (CT) scans, transcranial magnetic stimulation (TMS) and magnetic resonance imaging (MRI).

While recent advances in emergency care and rehabilitation allow many SCI patients to survive, methods for reducing the extent of injury and for restoring function are still limited. Acute treatment for SCI includes techniques to relieve cord compression, drug therapy with corticosteroids such as methylprednisolone to minimize cell damage, and stabilization of the vertebrae of the spine to prevent further injury.
WO 2010/111221 A2 which corresponds to US 2010/0247677 discloses compositions for preferential distribution of active agents to injury sites comprising a delivery ligand and one or more active agents, wherein the delivery ligand is PEG having a molecular weight of 300-9000 Da in an amount of at least 20% w/v and wherein the active agent is magnesium, optionally in the form of magnesium chloride hexahydrate, in a range of about 0.1-20% w/v. The compositions have been used in a spinal cord injury model study in rats.
WO 2010/111215 A3 which corresponds to US 2010/0247676 discloses compositions and methods for treatment of hemorrhage, the compositions comprising a vessel closing compound which may comprise a polymer with hydrophilic properties such as PEG, and also one or more active agents such as a blood flow modifier with a potential to form ionic bonds with the vessel closing agent. The use of composition is disclosed comprising 0.8% magnesium in 20 or 30% PEG3350, or PEG3000-biotin, in 1-5 single infusions, in a model study of the treatment of spinal cord injury in rats.
Jae H.T. Lee et al, "Magnesium in a polyethylene glycol formulation provides neuroprotection after unilateral cervical spinal cord injury", SPINE 15:2041-2048 (Nov. 2010), disclose an experimental animal study to investigate the neuroprotective efficacy of magnesium in PEG formulation in a contusive model of cervical spinal cord injury (SPI). It was found that MgCl₂ in a PEG formulation reduced secondary damage and improved behavioral recovery when administered 2 hours after a unilateral cervical hemicontusion injury.
Brian K. Kwon et al., "Magnesium chloride in a polyethylene glycol formulation as a neuroprotective therapy for acute spinal cord injury: preclinical refinement and optimization", Journal of Neurotrauma 26:1379-1393 (Aug. 2009) disclose a series of experiments evaluating the neuroprotective efficacy of magnesium in a PEG formulation using an acute model of thoracic SCI. The goal of these experiments was to help guide the treatment parameters for a clinical trial of magnesium within a PEG formulation in acute human SCI.

Despite vigorous research into the use of anti-inflammatory drugs, ionizing irradiation, elimination of inhibitory factors in the spinal cord, provision of neurotrophic factors, and cell transplantation, there currently is no sufficient treatment for SCI.

Therefore, there is a need to develop new methods and compositions for the treatment of neuronal injury including spinal cord injury.

### SUMMARY

According to the present invention a composition is provided for use in the treatment of a neuronal injury in a patient suffering therefrom, the composition comprising an injectable solution of magnesium, polyethylene glycol and an anti-oxidant, wherein the magnesium is magnesium chloride hexahydrate, the polyethylene glycol is polyethylene glycol 3350 (PEG3350) and the anti-oxidant comprises butylated hydroxyltoluene (BHT), wherein the magnesium chloride hexahydrate and PEG3350 are packaged separately and then combined before administration, wherein the magnesium chloride hexahydrate is supplied in a first vial comprising an aqueous solution of magnesium where the magnesium is administered in a dosing range from about 0.01 to about 0.5 mmol of Mg ions/kg, and the PEG3350 together with the anti-oxidant comprising BHT is supplied in a second vial comprising an aqueous solution containing PEG3350 and sodium chloride where the PEG3350 is administered in a dosing range at about 0.1 to about 60% w/v, w/w and/or v/v, and the anti-oxidant comprising BHT is in an amount of about 0.00001% to about 5% w/v/, w/w and/or v/v of the composition, wherein at least three doses of the composition are administered to the patient. Note that the invention is defined by the claims. Any subject-matter falling outside the scope of the claims is provided for information purposes only. Any references in the description to methods of treatment refer to the compounds, pharmaceutical compositions and medicaments of the present invention for use in a method for treatment of the human (or animal) body by therapy.

The instant disclosure fulfills this and the other foregoing needs by providing compositions and methods for treatment of conditions associated with neuronal injury (e.g., spinal cord injury). In some embodiments, the spinal cord injury can be acute traumatic spinal cord injury with no evidence of penetrating or transection injury (e.g., caused by projectile or stab wound).

In various embodiments, the present disclosure provides a method of treating a patient suffering from a neuronal injury. The method comprises administering to the patient at least three doses of a first composition comprising magnesium in an amount of between about 0.1% to about 30% w/w, w/v, or v/v of the first composition and at least three doses of a second composition comprising polyethylene glycol (PEG) in an amount of from about 10% to about 60% w/w, w/v, or v/v of the second composition.

In some embodiments, a method of treating a patient suffering from a neuronal injury (e.g., spinal cord injury) is provided. The method comprises administering to the patient at least three doses of a first composition comprising magnesium in an amount of between about 1 gram to about 50 grams in the first composition and at least three doses of a second composition comprising polyethylene glycol in an amount of from about 10 grams per 100 milliliters to about 60 grams per 100 milliliters of the second composition.

In various embodiments, a method of treating a patient suffering from a neuronal injury is provided. The method comprises administering to the patient at least three doses of a composition comprising magnesium in an amount of between about 0.1% to about 30% w/w, w/v, or v/v of the composition and polyethylene glycol in an amount of from about 10% to about 60% w/w, w/v, or v/v of the composition.

In some embodiments, a method of treating a patient suffering from a neuronal injury is provided. The method comprises administering to the patient at least three doses of a composition comprising magnesium in an amount of between about 1 gram to about 50 grams and polyethylene glycol in an amount of from about 10 grams per 100 milliliters to about 60 grams per 100 milliliters of the composition.

In various embodiments, a kit for treating a patient suffering from a neuronal injury is provided. The kit comprises at least three doses of a first composition comprising magnesium in an amount of between about 0.1% to about 30% w/w, w/v, or v/v of the first composition and at least three doses of a second composition comprising polyethylene glycol in an amount of from about 10% to about 60% w/w, w/v, or v/v of the second composition.

In some embodiments, a kit for treating a patient suffering from a neuronal injury is provided. The kit comprises at least three doses of a first composition comprising magnesium in an amount of between about 1 gram to about 50 grams in the first composition and at least three doses of a second composition comprising polyethylene glycol in an amount of from about 10 grams per 100 milliliters to about 60 grams per 100 milliliters of the second composition.

In various embodiments, a kit for treating a patient suffering from a neuronal injury is provided. The kit comprises at least three doses of a composition comprising magnesium in an amount of between about 0.1% to about 30% w/w, w/v, or v/v of the composition and polyethylene glycol in an amount of from about 10% to about 60% w/w, w/v, or v/v in the composition.

In some embodiments, a kit for treating a patient suffering from a neuronal injury is provided. The kit comprises at least three doses of a composition comprising magnesium in an amount of between about 1 gram to about 50 grams and polyethylene glycol in an amount of from about 10 grams per 100 milliliters to about 60 grams per 100 milliliters in the composition.

In various embodiments, a method for treating a patient suffering from neuronal injury is provided. The method comprises administering to the patient at least six doses of a first composition comprising magnesium in an amount of between about 0.1% to about 30% w/w, w/v, or v/v of the first composition and at least six doses of a second composition comprising polyethylene glycol in an amount of from about 10% to about 60% w/w, w/v, or v/v of the second composition.

Additional features and advantages of various embodiments will be set forth in part in the description that follows, and in part will be apparent from the description, or may be learned by practice of various embodiments. The objectives and other advantages of various embodiments will be realized and attained by means of the elements and combinations particularly pointed out in the description and appended claims.

### BRIEF DESCRIPTION OF THE DRAWINGS

In part, other aspects, features, benefits and advantages of the embodiments will be apparent with regard to the following description, appended claims and accompanying drawings where:
Figure 1 depicts a graph showing preferential distribution of magnesium into the cerebrospinal fluid (CSF) following parenteral administration of magnesium in a PEG formulation relative to a magnesium in saline formulation and a saline formulation initiated after a neuronal injury (e.g., spinal cord injury);
Figure 2 illustrates accumulation and retention of biotin labeled PEG within the hemorrhagic site over a period of time after injury;
Figure 3 is a graphic illustration showing time-to-treatment initiation following spinal cord injury;
Figure 4 is a graphic illustration showing time-to-treatment initiation following traumatic brain injury;
Figure 5 depicts a chart showing the dosing used in healthy human volunteers that were administered saline, PEG and magnesium or MgSO4 formulations; and
Figure 6 provides a graph showing magnesium serum levels in mmol/L during different post-infusion time periods following parenteral administration of magnesium in a PEG formulation relative to a MgSO4 formulation and a saline formulation in healthy volunteers.

It is to be understood that the figures are not drawn to scale. Further, the relation between objects in a figure may not be to scale, and may in fact have a reverse relationship as to size. The figures are intended to bring understanding and clarity to the structure of each object shown, and thus, some features may be exaggerated in order to illustrate a specific feature of a structure.

### DETAILED DESCRIPTION

The instant disclosure provides compositions, methods and kits for treatment of conditions associated with neuronal injury. The combination of polyethylene (PEG) and magnesium is a significant development as it improves efficacy for the treatment of neuronal injury (e.g., spinal cord injury).

In some embodiments, the combination of polyethylene (PEG) and magnesium can be used in the management of acute spinal cord injury for improving neurological recovery and/or reducing the incidence of disability.

For the purposes of this specification and appended claims, unless otherwise indicated, all numbers expressing quantities of ingredients, percentages or proportions of materials, reaction conditions, and other numerical values used in the specification and claims, are to be understood as being modified in all instances by the term "about." Accordingly, unless indicated to the contrary, the numerical parameters set forth in the following specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements. Moreover, all ranges disclosed herein are to be understood to encompass any and all subranges subsumed therein. For example, a range of "1 to 10" includes any and all subranges between (and including) the minimum value of 1 and the maximum value of 10, that is, any and all subranges having a minimum value of equal to or greater than 1 and a maximum value of equal to or less than 10, *e.g.,* 5.5 to 10.

### Definitions

To aid in the understanding of the disclosure, the following non-limiting definitions are provided:
It is noted that, as used in this specification and the appended claims, the singular forms "a," "an," and "the," include plural referents unless expressly and unequivocally limited to one referent. Thus, for example, reference to "a composition" includes one, two, three or more compositions.

The term "therapeutic agent" as used herein is generally meant to refer to any substance that alters the physiology of a patient. The term "therapeutic agent" may be used interchangeably herein with the term "drug". It will be understood that unless otherwise specified a "therapeutic" formulation may include more than one therapeutic agent, wherein exemplary combinations of therapeutic agents include a combination of two or more drugs. The drug provides a concentration gradient of the therapeutic agent for delivery to the site.

A "therapeutically effective amount" or "effective amount" is such that when administered, the drug results in alteration of the biological activity, such as, for example, inhibition of inflammation, reduction or alleviation of pain, or improvement in the condition through neuroprotection, *etc.* The dosage administered to a patient can be as single or multiple doses depending upon a variety of factors, including the drug's administered pharmacokinetic properties, the route of administration, patient conditions and characteristics (sex, age, body weight, health, size, *etc*.), extent of symptoms, concurrent treatments, frequency of treatment and the effect desired.

"Treating" or "treatment" of a disease or condition refers to executing a protocol that may include administering one or more drugs to a patient (human, other normal or otherwise or other mammal), in an effort to alleviate signs or symptoms of the neuronal injury. Alleviation can occur prior to signs or symptoms of neuronal injury appearing, as well as after their appearance. In addition, treating or treatment does not require complete alleviation of signs or symptoms, does not require a cure, and specifically includes protocols that have only a marginal effect on the patient. "Reducing pain and/or inflammation" includes a decrease in pain and/or inflammation and does not require complete alleviation of pain and/or inflammation signs or symptoms, and does not require a cure. In various embodiments, reducing pain and/or inflammation includes even a marginal decrease in pain and/or inflammation. In various embodiments, the composition comprising PEG and magnesium improves sensory motor functions after, for example, stroke, traumatic brain injury, spinal cord, cranial nerve and/or peripheral nerve injuries. In various embodiments, after administration of the composition, improvement in sensory and/or motor functions is at about 5, 10, 15, 20, 25, 30, 35, 40, 45, 50, 55, 60, 65, 70, 75, 80, 85, 90, 95, 96, 97, 98 or 99% improvement. In some embodiments, improvement in sensory and/or motor function is measured by International Standards for Neurological Classification of Spinal Cord Injury, developed by ASIA and the International Spinal Cord Society (ISCoS). In some embodiments, the sensory and/or motor functions can include improvement in one or more autonomic functions such as, but not limited to, blood pressure regulation, bladder function, bowel function, sexual function and/or the like.

"Localized" delivery includes delivery where one or more drugs are deposited within a tissue, for example, a nerve root of the nervous system or a region of the brain, or in close proximity (within about 0.1 cm, or preferably within about 10 cm, for example) thereto. For example, the drug dose delivered locally may be, for example, 10%, 20%, 30%, 40%, 50%, 60%, 70%, 80%, 90%, 95%, 99%, or 99.9% less than the oral dosage or injectable dose. In turn, systemic side effects, such as for example, liver transaminase elevations, hepatitis, liver failure, myopathy, constipation, *etc.* may be reduced or eliminated.

The term "mammal" refers to organisms from the taxonomy class "mammalian," including humans, other primates such as chimpanzees, apes, orangutans and monkeys, rats, mice, cats, dogs, cows, horses, *etc.*

The term "patient" or "subject" includes a living or cultured system upon which the methods and/or kits of the current disclosure is used. The term includes, humans, or other mammals.

The term "practitioner" means a person who practices methods, kits, and compositions of the instant disclosure on the subject. The term includes, doctors, other medical personnel, and researchers.

The term "parenteral" as used herein refers to modes of administration that bypass the gastrointestinal tract, and include for example, intravenous, intramuscular, continuous or intermittent infusion, intraperitoneal, intrasternal, subcutaneous, intra-operatively, intrathecally, intradiscally, peridiscally, epidurally, perispinally, intraarticular injection or combinations thereof. In some embodiments, the injection is intrathecal, which refers to an injection into the spinal canal (intrathecal space surrounding the spinal cord). An injection may also be into a muscle or other tissue.

The term "neuronal injury" refers to an insult to an element of the central or peripheral nervous systems. Neuronal injuries can be derived from a physical (including mechanical, electrical or thermal), ischemic, hemorrhagic, chemical, biological or biochemical insult. Examples of neuronal injuries include, ischemic and hemorrhagic stroke, spinal cord (e.g., acute spinal cord injury), brain, traumatic brain, cranial nerve and peripheral nerve injuries.

The term "delivery vehicle" refers to a substance that is used to deliver the therapeutic agent. The delivery vehicle can also have beneficial properties.

All references to chemical compounds, including magnesium and polyethylene glycol, include all forms of these chemical compounds (e.g., salts, esters, hydrates, ethanolates, *etc*.), wherein said forms possess at least partial activities of the respective chemical compounds.

Reference will now be made in detail to certain embodiments of the disclosure, examples of which are illustrated in the accompanying drawings. While the disclosure will be described in conjunction with the illustrated embodiments, it will be understood that they are not intended to limit the disclosure to those embodiments. On the contrary, the disclosure is intended to cover all alternatives, modifications, and equivalents that may be included within the disclosure as defined by the appended claims.

It will be understood that the section headings below can be interchanged with other section headings.

### Neuronal Injury

Neuronal injuries are complex clinical conditions aggravated by a variety of precipitating causes that influence the severity of injury and ultimately influence the course and extent of recovery. A primary insult to a component of the central and/or peripheral nervous system could be of mechanical, chemical, biological or electrical nature. Following the primary insult, a cascade of biochemical and physiological events takes place that often leads to pathobiological changes that are considered largely responsible for the development of irreversible damages. This autodestructive cascade is known as secondary injury and because it develops over time after the traumatic event it opens a window of opportunity for pharmacological interventions. Various chronic conditions linked to persistent on-going tissue damage due, for example, to inflammatory reactions or autoimmune diseases, may also lead to secondary injury of neuronal components and symptomatic pain. Neuronal injuries include spinal cord (e.g., acute spinal cord injury).

There are several classes of events that are determinant in the secondary phase of the traumatic brain injury (TBI) pathology and other neuronal injuries. One of these is membrane damages to cells that managed to survive the first impact. Small changes in membrane integrity and/or cytoskeletal architecture can impair membrane potential, intracellular transport and ATP production leading to cytoskeletal collapse, mitochondrial dysfunction, energetic failure and free radical production resulting in cell death by either apoptotic or necrotic mechanisms. In some instances, the dying cells can release free radicals and catabolic enzymes (proteinase, peptidases, caspases) that may cause damages to the surrounding cells and increase the number of injured cells. Unfortunately, neuronal cells are particularly vulnerable to membrane damages due to their high energetic demand and their unique anatomical structure which increase by many folds the challenge of maintaining efficient membrane integrity and intracellular (axonal) transport.

Another class of events that plays a major role in the secondary phase of neuronal injuries is the storm of neurotransmitter release which by mechanisms globally referred as "excitotoxicity" which can increase the vulnerability of neurons to any additional insults in an area that, in size, extend far beyond the area directly affected by the first insult. For example, a marked increase in extracellular glutamate levels is often associated with neuronal insults. Since glutamate is the most prominent excitatory neurotransmitter of the central nervous system, almost all neurons have glutamate receptors and will be affected by the toxic events triggered by excessive amounts of extracellular glutamate. Excitotoxicity insults are thought to be largely triggered by excessive inflow of Ca²⁺ through a specific subtype of glutamate receptor, the N-methyl-D-aspartate receptors (NMDAR). High concentrations of intracellular Ca²⁺ can activate catabolic enzymes and production of free radicals that may interfere with the repair mechanisms of the cell or its ability to cope with additional challenges or even precipitate cell death.

In some embodiments, the neuronal injury comprises a spinal cord injury. In some embodiments, the neuronal injury comprises ischemic injury. In some embodiments, the neuronal injury comprises stroke including hemorrhagic stroke, ischemic stroke, or the like. In some embodiments, the neuronal injury comprises brain injury. In some embodiments, the neuronal injury comprises traumatic brain injury. In some embodiments, the neuronal injury comprises cranial nerve injury. In some embodiments, the neuronal injury comprises peripheral nerve injury.

### Polyethylene Glycol

For more than 40 years, biomembrane sealing agents of various molecular weights have been utilized as adjuncts to culture media for their ability to protect cells against fluid-mechanical injuries. These agents include hydrophilic polymers such as polyoxyethylenes, polyalkylene glycol, polyethylene glycols (PEG), polyvinyl alcohol, amphipatic polymers such as pluronics or poloxamers, including poloxamer P-188 (also known as CRL-5861, available from CytRx Corp., Los Angeles, Calif.) (Michaels and Papoutsakis, 1991) as well as methyl cellulose (Kuchler et al., 1960), sodium carboxylmethyl cellulose, hydroxyethyl starch, polyvinyl pyrrolidine and dextrans (Mizrahi and Moore, 1970; Mizrahi, 1975; Mizrahi, 1983).

Some biomembrane sealing agents including hydroxyethyl starch (Badet et al., 2005) and PEG (Faure et al, 2002; Hauet et al., 2001) have shown effective cryopreservative abilities in organ transplantation studies. Poloxamer P-188 was shown to protect articular cells from secondary injury following mechanical trauma to knee joint which could lead to acute pain and inflammation and potentially develop into a more chronic condition known as osteoarthritis (Phillips and Haut, 2004). Poloxamer P-188 and a neutral dextran protected muscle cells against electroporation or thermally driven cell membrane permeabilization (Lee et al., 1992). Direct application of PEG was shown to anatomically and functionally reconnect transected or crushed axon (Bittner et al., 1986), peripheral nerve (Donaldson et al., 2002) and spinal cord preparations in vitro (Lore et al., 1999; Shi et al., 1999; Shi and Borgens, 1999; Shi and Borgens, 2000; Luo et al., 2002) or in vivo (Borgens et al., 2002). Intravenous or subcutaneous administration of PEG or Poloxamer P-188 improved the cutaneous trunchi muscle reflex response after experimental spinal cord contusion in guinea pigs (Borgens and Bohnert, 2001; Borgens et al., 2004) and improved functional recovery in a naturally occurring spinal cord injury model in dogs (Laverty et al., 2004). PEGs of various molecular weights from 1,400-20000 Da, having a linear or multiple arms structure were shown to improve recovery following tissue injury (Hauet et al., 2001; Detloff et al., 2005; Shi et al., 1999).

PEG has been administered to treat neuronal injury as described in U.S. Patent Application No. 11/418,152 filed May 3, 2006, U.S. Application Publication No. 2007/0259044; U.S. Patent Application No. 13/495,561 filed June 13, 2012, published as U.S. Application Publication No. 2012/0302543; U.S. Patent Application No. 13/236,579 filed September 19, 2011, published as U.S. Application Publication No. 2012/0296112; U.S. Patent Application No. 13/574,105 filed January 21, 2011 published as U.S. Application Publication No. 2012/0295935 and U.S. Patent Application No. 13/552,530 filed July 18, 2012, published as U.S. Application Publication No. 2012/0283450.

PEG can be effective following different modes of delivery including local and prolonged cellular exposure, direct and short-term tissue or organ exposure or systemic administration. Effective concentrations of biomembrane fusion agents may vary depending on the purpose and/or mode of delivery. For example, about 0.05% concentration is effective in tissue culture applications (Michaels and Papoutsakis, 1991) and about 30% to about 50% concentration is effective for organ preservation and upon in vivo administration in animals (Hauet et al., 2001; Shi et al., 1999; Borgens and Bohnert, 2001; Borgens et al., 2004).

In some embodiments, PEG is administered at about 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, 15.0, 15.5, 16.0, 16.5, 17.0, 17.5, 18.0, 18.5, 19.0, 19.5, 20.0, 20.5, 21.0, 21.5, 22.0, 22.5, 23.0, 23.5, 24.0, 24.5, 25.0, 25.5, 26.0, 26.5, 27.0, 27.5, 28.0, 28.5, 29.0, 29.5, 30.0, 30.5, 31.0, 31.5, 32.0, 32.5, 33.0, 33.5, 34.0, 34.5, 35.0, 35.5, 36.0, 36.5, 37.0, 37.5, 38.0, 38.5, 39.0, 39.5, 40.0, 40.5, 41.0, 41.5, 42.0, 42.5, 43.0, 43.5, 44.0, 44.5, 45.0, 45.5, 46.0, 46.5, 47.0, 47.5, 48.0, 48.5, 49.0, 49.5, 50.0, 50.5, 51.0, 51.5, 52.0, 52.5, 53.0, 53.5, 54.0, 54.5, 55.0, 55.5, 56.0, 56.5, 57.0, 57.5, 58.0, 58.5, 59.0, 59.5 to about 60% w/v, w/w and/or v/v of the composition.

In some embodiments, PEG is administered at about 0.10, 0.11, 0.12, 0.13, 0.14, 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.33, 0.34, 0.35, 0.36, 0.37, 0.38, 0.39, 0.40, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 071, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, 15.0, 15.5, 16.0, 16.5, 17.0, 17.5, 18.0, 18.5, 19.0, 19.5, 20.0, 20.5, 21.0, 21.5, 22.0, 22.5, 23.0, 23.5, 24.0, 24.5, 25.0, 25.5, 26.0, 26.5, 27.0, 27.5, 28.0, 28.5, 29.0, 29.5, 30.0, 30.5, 31.0, 31.5, 32.0, 32.5, 33.0, 33.5, 34.0, 34.5, 35.0, 35.5, 36.0, 36.5, 37.0, 37.5, 38.0, 38.5, 39.0, 39.5, 40.0, 40.5, 41.0, 41.5, 42.0, 42.5, 43.0, 43.5, 44.0, 44.5, 45.0, 45.5, 46.0, 46.5, 47.0, 47.5, 48.0, 48.5, 49.0, 49.5 50.0, 50.5, 51.0, 51.5, 52.0, 52.5, 53.0, 53.5, 54.0, 54.5, 55.0, 55.5, 56.0, 56.5, 57.0, 57.5, 58.0, 58.5, 59.0, 59.5 to about 60.0 grams (g)/kg of the composition.

In some embodiments, the PEG in the above mentioned grams or w/v, w/w, and/or v/v% can be administered at one or more dose(s) of 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, 15.0, 15.5, 16.0, 16.5, 17.0, 17.5, 18.0, 18.5, 19.0, 19.5, 20.0, 20.5, 21.0, 21.5, 22.0, 22.5, 23.0, 23.5, 24.0, 24.5, 25.0, 25.5, 26.0, 26.5, 27.0, 27.5, 28.0, 28.5, 29.0, 29.5, 30.0, 30.5, 31.0, 31.5, 32.0, 32.5, 33.0, 33.5, 34.0, 34.5, 35.0, 35.5, 36.0, 36.5, 37.0, 37.5, 38.0, 38.5, 39.0, 39.5, 40.0, 40.5, 41.0, 41.5, 42.0, 42.5, 43.0, 43.5, 44.0, 44.5, 45.0, 45.5, 46.0, 46.5, 47.0, 47.5, 48.0, 48.5, 49.0, 49.5, 50.0, 55.0, 60.0, 65.0, 70.0, 75.0, 80.0, 85.0, 90.0, 95.0, 100.0, 105.0, 110.0, 115.0, 120.0, 125.0, 130.0, 135.0, 140.0, 145.0, 150.0, 155.0, 160.0, 165.0, 170.0, 175.0, 180.0, 185.0, 190.0, 195.0, 200.0, 200.5, 201.0, 201.5, 202.0, 202.5, 203.0, 203.5, 204.0, 204.5, 205.0, 205.5, 206.0, 206.5, 207.0, 207.5, 208.0, 208.5, 209.0, 209.5, 210.0, 210.5, 211.0, 211.5, 212.0, 212.5, 213.0, 213.5, 214.0, 214.5, 215.0, 215.5, 216.0, 216.5, 217.0, 217.5, 218.0, 218.5, 219.0, 219.5, 220.0, 220.5, 221.0, 221.5, 222.0, 222.5, 223.0, 223.5, 224.0, 224.5, 225.0, 225.5, 226.0, 226.5, 227.0, 227.5, 228.0, 228.5, 229.0, 229.5, 230.0, 230.5, 231.0, 231.5, 232.0, 232.5, 233.0, 233.5, 234.0, 234.5, 235.0, 235.5, 236.0, 236.5, 237.0, 237.5, 238.0, 238.5, 239.0, 239.5, 240.0, 240.5, 241.0, 241.5, 242.0, 242.5, 243.0, 243.5, 244.0, 244.5, 245.0, 245.5, 246.0, 246.5, 247.0, 247.5, 248.0, 248.5, 249.0, 249.5, 250.0, 255.0, 260.0, 265.0, 270.0, 275.0, 280.0, 285.0, 290.0, 295.0, 300.0, 300.5, 301.0, 301.5, 302.0, 302.5, 303.0, 303.5, 304.0, 304.5, 305.0, 305.5, 306.0, 306.5, 307.0, 307.5, 308.0, 308.5, 309.0, 309.5, 310.0, 310.5, 311.0, 311.5, 312.0, 312.5, 313.0, 313.5, 314.0, 314.5, 315.0, 316.0, 316.5, 317.0, 317.5, 318.0, 318.5, 319.0, 319.5, 320.0, 320.5, 321.0, 321.5, 322.0, 322.5, 323.0, 323.5, 324.0, 324.5, 325.0, 325.5, 326.0, 326.5, 327.0, 327.5, 328.0, 328.5, 329.0, 329.5, 330.0, 330.5, 331.0, 331.5, 332.0, 332.5, 333.0, 333.5, 334.0, 334.5, 335.0, 335.5, 336.0, 336.5, 337.0, 337.5, 338.0, 338.5, 339.0, 339.5, 340.0, 340.5, 341.0, 341.5, 342.0, 342.5, 343.0, 343.5, 344.0, 344.5, 345.0, 345.5, 346.0, 346.5, 347.0, 347.5, 348.0, 348.5, 349.0, 349.5, 350.0, 355.0, 360.0, 365.0, 370.0, 375.0, 380.0, 385.0, 390.0, 395.0 or 400.0 mg/kg of patient body weight (e.g., actual body weight or ideal body weight).

The PEG can be in the same composition with the magnesium or in a separate composition from the magnesium.

The PEG can be mixed with a suitable vehicle or a diluent and administered separately from the magnesium as a distinct composition or combined with the magnesium in one composition. In some embodiments, the delivery vehicle or diluent includes dextrose, other sugars including sucrose, fructose, glucose, lactated ringer's, polyols including mannitol, xylitol, sorbitol, maltitol, lactitol, polysaccharides including but not limited to native or pre-gelatinized starch, maltodextrins, cyclodextrins, mineral compounds including dicalcium or tricalcium phosphate, either dihydrate or anhydrous, cellulose derivatives including microcrystalline cellulose, lactoses either monohydrates thereof or anhydrous, as well as their mixtures such as dicalcium phosphate dihydrate, mannitol, pre-gelatinized maize starch, microcrystalline cellulose and their mixtures, water and/or NaCl (saline). In some embodiments, the saline is 0.90 saline or 0.45 saline. In some embodiments, other delivery vehicles can be used for example, D5W (dextrose in 5% water), D5NS (dextrose in 5% water and normal saline) and D5W/1/2NS (D5Wand ½ normal saline).

In some embodiments, PEG toxicity is measured by sampling bodily fluids, such as, for example, blood, urine or other bodily fluids and/or by the monitoring of physical symptoms.

In some embodiments, PEG has a molecular weight of from about 1,000 to about 10,000 Daltons. In various embodiments, PEG has a molecular weight of about 1,000 to about 4,000 Daltons. According to the invention, PEG has a molecular weight of about 3,350 Daltons.

### Magnesium

As discussed above, a combination of PEG and magnesium is useful for the treatment of neuronal injury. Magnesium plays an important role in a large diversity of cellular functions. For example, magnesium is required for glycolysis and oxidative phosphorylation which support energy-producing and energy-consuming reactions in cells. Protein synthesis as well as membrane structure and function are also magnesium-dependent. Levels of magnesium will affect neurotransmitter release including glutamate and acetylcholine release. It also regulates the activity of calcium transporters and opening of the non-methyl-D-aspartate (NMDA) glutamate receptors. Magnesium is known to have anti-oxidant, anti-apoptosis and to modulate lipid formation and transport. In addition to its cellular effects, magnesium can modulate physiological functions involved in regulation of blood flow and edema development.

During the last decade, a number of studies have reported that brain levels of free magnesium decline following TBI in animal model and in clinical settings. Decrease in brain levels of free magnesium from 40 to 60% has been observed in various TBI animal models including the fluid percussion model (Vink et al., 1991; Headrick et al., 1994), focal impact model (Suzuki et al., 1997) as well as more diffuse models of brain injury (Heath and Vink, 1996; Smith et al., 1998). Furthermore, in the rodent fluid percussion TBI model, a linear correlation was established between changes in brain free magnesium levels, energetic potential (phosphorylation potential) and functional (motor) outcomes (reviewed in Vink and Cernak, 2000). Decreases in magnesium levels have also been reported in experimental spinal cord injury (Vink et al., 1989).

A direct correlation was also established in TBI patients between levels of magnesium and the level of recovery (Mendez et al., 2005). TBI patients as well as human suffering from acute ischemic and/or cerebrovascular events are more susceptible to develop a condition called hypomagnesemia where availability of free magnesium is impaired (Polderman et al., 2000). Hypomagnesemia is also associated with increased mortality in patients in general who require the attention of the intensive care unit (Chernow et al., 1989; Rubeiz et al., 1993).

Magnesium supplementation initiated from minutes to hours after onset of CNS trauma showed neuroprotective effects in animal models of TBI (Heath and Vink, 1999; Esen et al., 2003; Vink et al., 2003; Feng et al., 2004 and Turner et al., 2004), spinal cord injury (Suzer et al., 1999; Kaptanoglu et al., 2003) and stroke (Yang et al., 2000; Westermaier et al., 2003 and 2005).

Clinical evaluation of intravenous administration of magnesium sulfate up to 12 hours following stroke onset showed no significant improvement in a multicenter trial involving 2589 patients (Muir et al., 2004). A follow-up study has been initiated to look at the potential effect of an earlier intervention where magnesium sulfate would be administered within 2 hours of stroke onset (Saver et al., 2004). Another clinical trial initiated in 1999 at the University of Washington (Seattle) was evaluating magnesium sulfate therapy for TBI patients and preliminary data were also negative in this trial.

Magnesium supplementation has also been extensively studied in animals and humans for its ability to reduce acute and chronic pain. However, mixed results have been reported from clinical trials evaluating the efficacy of magnesium (alone or in combination) in reducing pain associated with various surgical procedures (Bolcal et al., 2005; Apan et al., 2004; Bathia et al., 2004; McCartney et al., 2004), headache and acute migraine attacks (Cete et al., 2005; Corbo et al., 2001; Bigal et al, 2002), peripheral neuropathies (Brill et al., 2002; Felsby et al., 1996), cancer (Crosby et al., 2000), primary fibromyalgia syndrome (Moulin, 2001; Russel et al., 1995) and chronic limb pain (Tramer and Glynn, 2002). In addition, it appears that magnesium analgesic effect may be of a short duration such as 4 hours or less (Crosby et al., 2000). Magnesium may also induce side effects such as flushing and aching that can reduce its therapeutic window (Tramer and Glynn, 2002). Magnesium supplementation therapies can be achieved by using various salts including magnesium sulfate, chloride, gluconate and magnesium-ATP leading to similar neuroprotective effects in animal models of CNS injury (McIntosh et al., 1989; Izumi et al., 1991; Hoane et al., 2003; Turner et al., 2004; reviewed in Vink and McIntosh, 1990). It has been found that magnesium in combination with PEG has neuroprotective effects that are beneficial in the treatment of neuronal injury.

In some embodiments, the magnesium can be administered at about 0.01, 0.02, 0.03, 0.04, 0.05, 0.06, 0.07, 0.08, 0.09, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, 15.0, 15.5, 16.0, 16.5, 17.0, 17.5, 18.0, 18.5, 19.0, 19.5, 20.0, 20.5, 21.0, 21.5, 22.0, 22.5, 23.0, 23.5, 24.0, 24.5, 25.0, 25.5, 26.0, 26.5, 27.0, 27.5, 28.0, 28.5, 29.0, 29.5, 30.0, % w/v, w/w and/or v/v of the composition.

In some embodiments, the magnesium can be administered at about 0.10, 0.11, 0.12, 0.13, 0.14, 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.33, 0.34, 0.35, 0.36, 0.37, 0.38, 0.39, 0.40, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 071, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, 15.0, 15.5, 16.0, 16.5, 17.0, 17.5, 18.0, 18.5, 19.0, 19.5, 20.0, 20.5, 21.0, 21.5, 22.0, 22.5, 23.0, 23.5, 24.0, 24.5, 25.0, 25.5, 26.0, 26.5, 27.0, 27.5, 28.0, 28.5, 29.0, 29.5, 30.0, 30.5, 31.0, 31.5, 32.0, 32.5, 33.0, 33.5, 34.0, 34.5, 35.0, 35.5, 36.0, 36.5, 37.0, 37.5, 38.0, 38.5, 39.0, 39.5, 40.0, 40.5, 41.0, 41.5, 42.0, 42.5, 43.0, 43.5, 44.0, 44.5, 45.0, 45.5, 46.0, 46.5, 47.0, 47.5, 48.0, 48.5, 49.0, 49.5 to about 50.0 grams (g)/kg of the composition.

In some embodiments, the magnesium can be administered at one or more dose(s) of 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, 15.0, 15.5, 16.0, 16.5, 17.0, 17.5, 18.0, 18.5, 19.0, 19.5, 20.0, 20.5, 21.0, 21.5, 22.0, 22.5, 23.0, 23.5, 24.0, 24.5, 25.0, 25.5, 26.0, 26.5, 27.0, 27.5, 28.0, 28.5, 29.0, 29.5, 30.0, 30.5, 31.0, 31.5, 32.0, 32.5, 33.0, 33.5, 34.0, 34.5, 35.0, 35.5, 36.0, 36.5, 37.0, 37.5, 38.0, 38.5, 39.0, 39.5, 40.0, 40.5, 41.0, 41.5, 42.0, 42.5, 43.0, 43.5, 44.0, 44.5, 45.0, 45.5, 46.0, 46.5, 47.0, 47.5, 48.0, 48.5, 49.0, 49.5, 50.0, 55.0, 60.0, 65.0, 70.0, 75.0, 80.0, 85.0, 90.0, 95.0, 100.0, 105.0, 110.0, 115.0, 120.0, 125.0, 130.0, 135.0, 140.0, 145.0, 150.0, 155.0, 160.0, 165.0, 170.0, 175.0, 180.0, 185.0, 190.0, 195.0, 200.0, 200.5, 201.0, 201.5, 202.0, 202.5, 203.0, 203.5, 204.0, 204.5, 205.0, 205.5, 206.0, 206.5, 207.0, 207.5, 208.0, 208.5, 209.0, 209.5, 210.0, 210.5, 211.0, 211.5, 212.0, 212.5, 213.0, 213.5, 214.0, 214.5, 215.0, 215.5, 216.0, 216.5, 217.0, 217.5, 218.0, 218.5, 219.0, 219.5, 220.0, 220.5, 221.0, 221.5, 222.0, 222.5, 223.0, 223.5, 224.0, 224.5, 225.0, 225.5, 226.0, 226.5, 227.0, 227.5, 228.0, 228.5, 229.0, 229.5, 230.0, 230.5, 231.0, 231.5, 232.0, 232.5, 233.0, 233.5, 234.0, 234.5, 235.0, 235.5, 236.0, 236.5, 237.0, 237.5, 238.0, 238.5, 239.0, 239.5, 240.0, 240.5, 241.0, 241.5, 242.0, 242.5, 243.0, 243.5, 244.0, 244.5, 245.0, 245.5, 246.0, 246.5, 247.0, 247.5, 248.0, 248.5, 249.0, 249.5, 250.0, 255.0, 260.0, 265.0, 270.0, 275.0, 280.0, 285.0, 290.0, 295.0, 300.0, 305.0, 310.0, 315.0, 320.0, 325.0, 330.0, 335.0, 340.0, 345.0, 350.0, 355.0, 360.0, 365.0, 370.0, 375.0, 380.0, 385.0, 390.0, 395.0 or 400.0 mg/kg of patient body weight (e.g., actual body weight or ideal body weight).

The magnesium can be mixed with a suitable vehicle for delivery. In some embodiments, the delivery vehicle or diluent includes dextrose, other sugars including sucrose, fructose, glucose, lactated ringer's, polyols including mannitol, xylitol, sorbitol, maltitol, lactitol, polysaccharides including native or pre-gelatinized starch, maltodextrins, cyclodextrins, mineral compounds including dicalcium or tricalcium phosphate, either dihydrate or anhydrous, cellulose derivatives including microcrystalline cellulose, lactoses either monohydrates thereof or anhydrous, as well as their mixtures such as dicalcium phosphate dihydrate, mannitol, pre-gelatinized maize starch, microcrystalline cellulose and their mixtures, water and/or NaCl (saline). In some embodiments, the saline is 0.90% saline or 0.45% saline.

The magnesium can be administered in a separate composition from the PEG or it can be part of the same composition as the PEG.

In some embodiments, magnesium is measured by sampling bodily fluids, such as, for example, blood, urine, lymph or other bodily fluids and/or by the monitoring of physical symptoms.

It has been found that administration of PEG alone or magnesium alone had no effect on the loss of cognitive functions following brain injury whereas cognitive functions or more precisely, the ability to learn a new spatial task, was improved by >30% in animals treated with both PEG and magnesium solutions. Combined treatment with PEG and magnesium was also significantly more potent than treatment with either component alone in animal models of SCI reducing the lesion size by half, improving locomotor recovery and reducing the occurrence of neuropathic pain. In an acute model of tissue inflammation, the combined PEG and magnesium solution was also more effective than PEG or magnesium alone at reducing symptomatic pain. The discovery of the effect between PEG, and magnesium is highly significant as it can lead to the development of therapeutic formulations with improved efficacy for the treatment of neuronal trauma, inflammatory and painful conditions (U.S. Application Serial No. 11/418,153 filed May 3, 2006, published as U.S. Application Publication No. 2007/0258938,).

Neuronal improvement is shown when the subject demonstrates improvement at about 5%, 10%, 15%, 20%, 25%, 30%, 35%, 40%, 45%, 50% or more. In various embodiments, diagnostic techniques employed to monitor improvement are X-rays, computed tomography (CT) scans, transcranial magnetic stimulation (TMS) and magnetic resonance imaging (MRI).

These results suggest that PEG may also potentiate the beneficial effects of other therapeutic agents. According to the invention, the PEG, magnesium or a combination thereof contains an antioxidant.

Suitable examples of antioxidants include, free radical scavengers and chelators enzymes, co-enzymes, spin-trap agents, ion and metal chelators, lipid peroxidation inhibitors such as flavinoids, N-tert-butyl-alpha-phenylnitrone, NXY-059, Edaravone, glutathione and derivatives, and any combinations thereof.

Suitable examples of specific antioxidants include, without limitation, ascorbic acid, methionine, dilhiocarbamate, butylated hydroxytoluene (BHT), polyphenolic bioflavonoids, decosahexanoic acid (DHA), butylated hydroxyanisole (BHA), ascorbyl palmitate, tocopherols, ascorbic acid isomers and/or derivatives, sulfurous acid salts, thiol derivatives, nordihydroguaiaretic acid and propyl gallate, acetyl cysteine, adipic acid, citric acid, cysteine, disoduim editic acid (EDTA), fumaric acid, gutamic acid, malic acid, sodium formaldehyde sulfoxylate, sodium metabisulfite, sodium sulfite, sodium thosulfate, tartaric acid, thioglycerol, thiourea and toluene sulfonic acid. According to the invention, the antioxidant is butylated hydroxytoluene (BHT).

The antioxidants can be administered to a patient at about 0.00001, 0.00005, 0.00010, 0.00015, 0.00020, 0.00025, 0.00030, 0.00035, 0.00040, 0.00045, 0.00050, 0.00055, 0.00060, 0.00065, 0.00070, 0.00075, 0.00080, 0.00085, 0.00090, 0.00095, 0.0010, 0.0015, 0.0020, 0.0025, 0.0030, 0.0035, 0.0040, 0.0045, 0.0050, 0.0055, 0.0060, 0.0065, 0.0070, 0.0075, 0.0080, 0.0085, 0.0090, 0.0095, 0.010, 0.015, 0.020, 0.025, 0.030, 0.035, 0.040, 0.045, 0.050, 0.055, 0.060, 0.065, 0.070, 0.075, 0.080, 0.085, 0.090, 0.095, 0.10, 0.15, 0.20, 0.25, 0.30, 0.35, 0.40, 0.45, 0.50, 0.55, 0.60, 0.65, 0.70, 0.75, 0.80, 0.85, 0.90, 0.95, 1.0, 1.5, 2.0, 2.5, 3.0, 3.5, 4.0, 4.5 to about 5% w/v, w/w and/or v/v of the composition.

Thus, a combined treatment comprising an administration of PEG disclosed above, and magnesium has a positive effect in improving functional recovery from neuronal injury.

The magnesium in the composition may be in salt form that is not toxic. Suitable examples of magnesium include magnesium nitrate, magnesium phosphate, magnesium carbonate, magnesium dihydrogenphosphate, magnesium oxalate, magnesium acetate, magnesium lactate, magnesium succinate, magnesium citrate, magnesium sulfate, magnesium sulfate heptahydrate, magnesium chloride, magnesium gluconate, magnesium ATP, magnesium oxide, magnesium hydroxide, magnesium aminobutyrate, magnesium arginate, magnesium aspartate, magnesium betainate, magnesium carnitinate, magnesium glycinate, magnesium lysinate, magnesium propionate, magnesium tartrate and any combination thereof. According to the invention, the magnesium is magnesium chloride hexahydrate.

In some embodiments, the composition comprises magnesium chloride. In some embodiments, the composition comprises magnesium chloride to decrease the toxicity and to increase the safety of the composition. In some embodiments, the composition comprises magnesium sulfate or other salts compatible with PEG.

A person of ordinary skill in the art will also appreciate that at least one marker may be included into the composition of the present disclosure. For example, the at least one marker may comprise any molecule or a cocktail of ingredients distribution of which is easy to visualize and monitor. Thus, in one embodiment, the at least one marker may be a radiographic marker, such as for example, barium, calcium phosphate, and metal beads. In another embodiment, the at least one marker may comprise iodine-based contrast agents, such as, for example, iopamidol, commercially available as Isovue™ (Bracco Diagnostics Inc., Princeton, N.J.) or iodixanol, commercially available as Visipaque™ (Nyocomed, Inc., Princeton, N.J.), and gandolinium-based contrast agents, such as, for example, gadodiaminde, commercially available as Omniscan™ (available from GE Healthcare, Princeton, N.J.). Therapeutic Formulations

Therapeutic formulations comprising the composition of the present disclosure can be prepared for storage by mixing PEG and magnesium with other agents, optional physiologically acceptable carriers, excipients or stabilizers (see, e.g., Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenyl, butyl or benzyl alcohol; alkyl parabens such as methyl or propyl paraben; catechol; resorcinol; cyclohexanol; 3-pentanol; and m-cresol); low molecular weight (less than about 10 residues) polypeptide; proteins, such as serum albumin, gelatin, or immunoglobulins; amino acids such as glycine, glutamine, asparagine, histidine, arginine, or lysine; monosaccharides, disaccharides, and other carbohydrates including glucose, mannose, dextrose or dextrins; chelating agents such as EDTA; sugars such as sucrose, mannitol, trehalose or sorbitol; salt-forming counter-ions such as sodium; metal complexes (e.g., Zn-protein complexes) and/or water. In some embodiments, the composition provided may have no preservatives in it.

Therapeutic formulations comprising the pharmaceutical composition of the present disclosure can be delivered in a delivery vehicle or a diluent that includes dextrose, other sugars including sucrose (e.g. D5W), fructose, glucose, lactated ringer's, polyols including mannitol, xylitol, sorbitol, maltitol, lactitol, polysaccharides including native or pre-gelatinized starch, maltodextrins, cyclodextrins, mineral compounds including dicalcium or tricalcium phosphate, either dihydrate or anhydrous, cellulose derivatives including microcrystalline cellulose, lactoses either monohydrates thereof or anhydrous, as well as their mixtures such as dicalcium phosphate dihydrate, mannitol, pre-gelatinized maize starch, microcrystalline cellulose and their mixtures, water and/or NaCl (saline). In some embodiments, the saline is 0.90% saline or 0.45% saline.

The formulations herein may also contain more than one active compound as necessary for the particular indication being treated, preferably those with complementary activities that do not adversely affect each other. Such molecules are suitably present in combination in amounts that are effective for the purpose intended.

The PEG and/or the magnesium may also be entrapped in a microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may also be prepared. Suitable examples of sustained-release preparations include semipermeable matrices of solid polymers containing PEG and/or magnesium, which matrices are in the form of shaped articles, e.g., films, or microcapsule. Examples of sustained-release matrices include, polyesters, hydrogels (for example, poly(2-hydroxyethyl-methacrylate), or poly(vinylalcohol)), polylactides (see, e.g., U.S. Pat. No. 3,773,919), copolymers of L-glutamic acid and y ethyl-L-glutamate, nondegradable ethylene-vinyl acetate, degradable lactic acid-glycolic acid copolymers such as the lactic acid-glycolic acid copolymer and poly-D-(-)-3-hydroxybutyric acid. Polymers such as ethylene-vinyl acetate and lactic acid-glycolic acid enable release of molecules for over 100 days.

Further, a person of ordinary skill in the art will recognize that PEG and/or magnesium may be implanted into the subject, for example, via parenteral administration in the form of a pump that can be inserted at or near the target site (e.g., spinal injury), or intravenous infusion or injection.

Parenteral administration may additionally include, for example, through a catheter near the spine or one or more inflamed joints, an implantable controlled release device or sustained release delivery system that can release a certain amount of the statin per hour or in intermittent bolus doses.

A person of ordinary skill in the art will recognize that the use of the at least one marker is especially advantageous in combination with this embodiment of the disclosure. As discussed above, the at least one marker may be a radiographic marker, such as, for example, barium, calcium phosphate, and metal beads. In another embodiment, the at least one marker may comprise iodine-based contrast agents, such as, for example, iopamidol, commercially available as Isovue™ (Bracco Diagnostics Inc., Princeton, N.J.) or iodixanol, commercially available as Visipaque™ (Nyocomed, Inc., Princeton, N.J.), and gandolinium-based contrast agents, such as, for example, gadodiaminde, commercially available as Omniscan™ (available from GE Healthcare, Princeton, N.J.). Such markers will also permit the practitioner to track delivery of the PEG and magnesium to the target tissue site of the neuronal injury. Thus treatment can be monitored using diagnostic imaging procedures known to one of ordinary skill in the art. Such diagnostic imaging procedures include for example, X-ray imaging or fluoroscopy.

In another embodiment, the PEG and/or the magnesium may be administered locally via a catheter positioned at or near a site of the pathological condition, e.g., neuronal injury. In this embodiment, the catheter has a proximal end and a distal end, the proximal end having an opening to deliver the PEG and/or the magnesium in situ, the distal end being fluidly connected to a pharmaceutical delivery pump. For example, the proximal end of the catheter delivers the PEG and/or the magnesium within 10 cm of the site of the pathological condition, more particularly, within 5 cm of the site of the pathological condition, and even more particularly, within 1 cm of the site of the pathological condition. The catheter may be positioned via a minimally invasive procedure, such as, for example, by accessing a blood vessel adjacent or supplying blood to the site of the pathological condition.

It will be understood that the PEG and the magnesium may be delivered independently of each other. In one example, the PEG may be delivered through an intramuscular injection and the magnesium is delivered via an implant. A person of ordinary skill in the art will undoubtedly recognize that a large number of combinations is possible.

A person of ordinary skill in the art will further recognize that in some cases it may be advantageous to ship and store the PEG and the magnesium separately and pre-mix these compounds at a desired time, e.g., one hour prior to administration, or even to administer those compounds without pre-mixing. Accordingly, in another aspect, the disclosure provides a kit comprising PEG administered in a first delivery fluid, magnesium administered in a second delivery fluid, and a set of instructions comprising information on making an injectable composition. In various embodiments, the magnesium is magnesium chloride hexahydrate and the composition further includes at least one antioxidant. In some embodiments, the PEG, magnesium or a combination thereof in the composition is administered via a bolus infusion, a continuous infusion, an intravenous administration (IV), an intramuscular administration, an intrathecal administration, a subcutaneous administration, an epidural administration, a parenteral administration, an intra-articular administration, a direct application or deposition onto or adjacent to a site of the pathological condition, and any combinations thereof.

A person of ordinary skill in the art will further recognize that the kit provides a practitioner with an advantageous flexibility in selecting the ratios of PEG and the magnesium.

A person of ordinary skill in the art will appreciate that the set of instruction may be provided in any medium, including, printed, audio and video recorded, and electronic.

In another aspect, the disclosure provides a method of treating a neuronal injury, the method comprising administering to a subject in need thereof at least three doses of a therapeutically effective amount of PEG and at least three doses of a therapeutically effective amount of magnesium, wherein PEG and the magnesium are delivered in an injectable composition.

In various embodiments, the subject is administered at least six doses of a therapeutically effective amount of PEG and at least six doses of a therapeutically effective amount of magnesium. In some embodiments, two sets of three doses of a therapeutically effective amount of PEG and two sets of three doses of a therapeutically effective amount of magnesium is administered to the patient. In various embodiments, one dose of a therapeutically effective amount of PEG and one dose of a therapeutically effective amount of magnesium is administered to the patient for 30 minutes by or within 12 hours post injury.

In some embodiments, one dose of a therapeutically effective amount of PEG and one dose of a therapeutically effective amount of magnesium is administered to the patient for 30 minutes by or within 1, 2, 3, 4, 5, 6, 7, 8, 9, 10, 11 or 12 hours post injury (e.g., such as 6 or 12 hours post injury). In some embodiments, one dose of a therapeutically effective amount of PEG and one dose of a therapeutically effective amount of magnesium is administered by or within 1, 2, 3, 4, 5, 6, 7, 8, 9, 10, 11 or 12 hours post injury (e.g., such as 6 or 12 hours post injury). The PEG and magnesium can be in the same composition or in separate compositions.

In some embodiments, one dose of a therapeutically effective amount of PEG and/or one dose of a therapeutically effective amount of magnesium is administered to the patient by or within 1, 2, 3, 4, 5, 6, 7, 8, 9, 10, 11 or 12 hours post injury (e.g., such as 6 or 12 hours post injury). In some embodiments, one dose of a therapeutically effective amount of PEG and/or one dose of a therapeutically effective amount of magnesium is administered by or within 1, 2, 3, 4, 5, 6, 7, 8, 9, 10, 11 or 12 hours post injury (e.g., such as 6 or 12 hours post injury). The PEG and magnesium can be in the same composition or in separate compositions.

Following these first initial doses, the subject receives an additional 5 doses of the therapeutically effective amount of PEG and 5 additional doses of the therapeutically effective amount of magnesium. The 5 additional doses of the therapeutically effective amount of PEG and the 5 additional doses of the therapeutically effective amount of magnesium will each be administered during 6 hours intervals.

In some embodiments, one or more doses of the PEG and/or magnesium can be administered in 1, 2, 3, 4, 5, 6, 7, 8, 9, 10, 11 or 12 hour intervals.

In various embodiments, at least 1 to 24 doses of PEG and magnesium may be administered. In some embodiments, the magnesium dose will be given by or within the first 24 hours and a total magnesium dose in 30 hours. That is, the magnesium can be given in 1, 2, 3, 4, 5, 6, 7, 8, 9, 10, 11, 12, 13, 14, 15, 16, 17, 18, 19, 20, 21, 22, 23 or 24 doses. In some embodiments, the PEG can be given in 1, 2, 3, 4, 5, 6, 7, 8, 9, 10, 11, 12, 13, 14, 15, 16, 17, 18, 19, 20, 21, 22, 23 or 24 doses.

In some embodiments, one or more doses of PEG and/or magnesium are given by or within 1, 2, 3, 4, 5, 6, 7, 8, 9, 10, 11, 12, 13, 14, 15, 16, 17, 18, 19, 20, 21, 22, 23 or 24 hours post injury.

In some embodiments, the composition comprising PEG and magnesium can be administered in a dosing range from about 0.01 to about 0.5 mmol of Mg ions/kg. In some embodiments, the magnesium can be administered in a dose of about 0.01, 0.02, 0.03, 0.04, 0.05, 0.06, 0.07, 0.08, 0.09, 0.1, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.30, 0.31, 0.32, 0.33, 0.34, 0.35, 0.36, 0.37, 0.38, 0.39, 0.40, 0.41, 0.42, 0.43, 0.44, 0.45, 0.46, 0.47, 0.48, 0.49, 0.5, 0.6, 0.7, 0.8, 0.9, 1.0, 1.5, 2.0, 2.2, 2.4, 2.5, 2.6, 2.8, 3.0, 3.2, 3.4, 3.5, 3.8, 4.0, 4.2, 4.4, 4.5, 5.0, 5.5, 6.0, 6.5, 7.0, 7.5, 8.0, 8.5, 9.0, 9.5, 10.0, 10.5, 11.0, 11.5 mmol Mg ions/kg.

In some embodiments, the composition comprising PEG and magnesium can be administered in a dosing range from about 0.02 to about 0.2 mmol of Mg ions/kg. In some embodiments, the magnesium can be administered in a dose of about 0.02, 0.03, 0.04, 0.05, 0.06, 0.07, 0.08, 0.09, 0.1, 0.1, 0.11, 0.12, 0.13, 0.14, 0.15, 0.16, 0.17, 0.18, 0.19 and/or 0.2 mmol Mg ions/kg.

In some embodiments, a first dose of the composition comprising PEG and magnesium of up to 0.2 mmol of Mg ions/kg/infusion is administered and a total of 5 additional doses of the composition is administered. Each dose is delivered over 30 minutes via a pump-controlled infusion and separated by a 6-hour interval for a total dose of 1.2 mmol Mg ions/kg. In various embodiments, a fixed dose of the composition comprising PEG and magnesium of up to 103 mmol of Mg ions/kg over 30 hours is administered.

In some embodiments, the magnesium can be administered at a dose of from about 2.5, 3.0, 3.5, 3.9, 4.0, 4.5, 5.0, 5.5, 6.0, 6.5, 7.0, 7.5, 7.8, 8.0, 8.5, 9.0, 9.5, 10, 10.5, 11, 11.5, 12, 12.5, 13, 13.5, 14, 14.5, 15, 15.5, 15.6, 16, 16.5, 17, 17.5, 18, 18.5, 19, 19.5, 20, 20.5, 21, 21.5, 22, 22.5, 23, 23.4, 23.5, 25, 26, 27, 28, 29, 30, 31, 32, 33, 34, 35, 36, 37, 38. 38.5, 39, 39.5 , 40, 41, 42, 43, 44, 45, 46, 47, 48, 49, 50, 50.5, 51.0, 51.5, 52.0, 52.5, 53.0, 53.5, 54.0, 54.5, 55.0, 55.5, 56.0, 56.5, 57.0, 57.5, 58.0, 58.5, 59.0, 59.5, 60.0, 60.5, 61.0, 61.5, 62.0, 62.5, 63.0, 63.5, 64.0, 64.5, 65.0, 65.5, 66.0, 66.5, 67.0, 67.5, 68.0, 68.5, 69.0, 69.5 to about 70.0 mg/kg of patient body weight (e.g., actual patient body weight or ideal patient body weight) alone or in combination with the PEG in the same or different compositions. The dose can be decreased, increased or escalated or stay the same as treatment progresses. In some embodiments, the dose of magnesium can be 100, 150, 200, 250, 300, 350, 400, 450, 500, 550, 600, 650, 700, 750, 800, 850, 900, 950, 1000 mg/kg that can be given as a single dose, or as multiple doses (e.g., 2, 3, 4, 5, 6, 7, 8, 9, 10, 11, or 12 doses, etc.). In some embodiments, the dose of magnesium can be 200, 200.5, 201.0, 201.5, 202.0, 202.5, 203.0, 203.5, 204.0, 204.5, 205.0, 205.5, 206.0, 206.5, 207.0, 207.5, 208.0, 208.5, 209.0, 209.5, 210.0, 210.5, 211.0, 211.5, 212.0, 212.5, 213.0, 213.5, 214.0, 214.5, 215.0, 215.5, 216.0, 216.5, 217.0, 217.5, 218.0, 218.5, 219.0, 219.5, 220.0, 220.5, 221.0, 221.5, 222.0, 222.5, 223.0, 223.5, 224.0, 224.5, 225.0, 225.5, 226.0, 226.5, 227.0, 227.5, 228.0, 228.5, 229.0, 229.5, 230.0, 230.5, 231.0, 231.5, 232.0, 232.5, 233.0, 233.5, 234.0, 234.5, 235.0, 235.5, 236.0, 236.5, 237.0, 237.5, 238.0, 238.5, 239.0, 239.5, 240.0, 240.5, 241.0, 241.5, 242.0, 242.5, 243.0, 243.5, 244.0, 244.5, 245.0, 245.5, 246.0, 246.5, 247.0, 247.5, 248.0, 248.5, 249.0, 249.5, 250.0, 255.0, 260.0, 265.0, 270.0, 275.0, 280.0, 285.0, 290.0, 295.0, 300.0 mg/kg that can be given as a single dose, or as multiple doses (e.g., 2, 3, 4, 5, 6, 7, 8, 9, 10, 11, or 12 doses, etc.).

In some embodiments, the ratio of PEG to magnesium in a single and/or separate composition can be at about 0.5 to about 40. In some the ratio of PEG to magnesium in a single or separate composition can be at about 1:39, 2:38, 3:37, 4:36, 5:35, 6:34, 7:33, 8:32, 9:31, 10:30, 11:29, 12:28, 13:27, 14:26, 15:25, 16:24, 17:23, 18:22, 19:21, 20:20, 21:19, 22:18, 23:17, 24:16, 25:15, 26:14, 27:13, 28:12, 29:11, 30:10, 31:9, 32:8, 33:7, 34:6, 35:5, 36:4, 37:3, 38:2, 39:1 or about 40:0.5.

In some embodiments, the magnesium can be administered in a separate composition from the PEG or it can be part of the same composition as the PEG. In some embodiments, a magnesium composition can be administered concurrently with a PEG composition (e.g., PEG composition, and magnesium composition being administered at the same time) or first the magnesium composition, then the PEG composition can be administered or first the PEG composition, then the magnesium composition can be administered. In some embodiments, the PEG and magnesium can be packaged separately and then combined before administration. In various embodiments, the PEG and magnesium can be package together as a single composition.

In some embodiments, the PEG and magnesium can be packaged together as a single composition in a glass vial. In some embodiments, the magnesium and the PEG are each supplied in separate glass vials to be mixed together prior to delivery. In various embodiments, the first glass vial comprises an aqueous solution containing magnesium and the second vial comprises an aqueous solution containing PEG and sodium chloride (e.g., 0.9% NaCl, 0.45% NaCl). In some embodiments, the container comprises an aqueous solution containing magnesium chloride hexahydrate and the second vial comprises an aqueous solution containing PEG and sodium chloride.

In various embodiments, the container comprises an aqueous solution of magnesium where the magnesium can be administered at about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, 15.0, 15.5, 16.0, 16.5, 17.0, 17.5, 18.0, 18.5, 19.0, 19.5, 20.0, 20.5, 21.0, 21.5, 22.0, 22.5, 23.0, 23.5, 24.0, 24.5, 25.0, 25.5, 26.0, 26.5, 27.0, 27.5, 28.0, 28.5, 29.0, 29.5, 30.0, % w/v, w/w and/or v/v.

In some embodiments, the first glass vial comprises an aqueous solution of magnesium where the magnesium can be administered at about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.33, 0.34, 0.35, 0.36, 0.37, 0.38, 0.39, 0.40, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 071, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, 15.0, 15.5, 16.0, 16.5, 17.0, 17.5, 18.0, 18.5, 19.0, 19.5, 20.0, 20.5, 21.0, 21.5, 22.0, 22.5, 23.0, 23.5, 24.0, 24.5, 25.0, 25.5, 26.0, 26.5, 27.0, 27.5, 28.0, 28.5, 29.0, 29.5, 30.0, 30.5, 31.0, 31.5, 32.0, 32.5, 33.0, 33.5, 34.0, 34.5, 35.0, 35.5, 36.0, 36.5, 37.0, 37.5, 38.0, 38.5, 39.0, 39.5, 40.0, 40.5, 41.0, 41.5, 42.0, 42.5, 43.0, 43.5, 44.0, 44.5, 45.0, 45.5, 46.0, 46.5, 47.0, 47.5, 48.0, 48.5, 49.0, 49.5 to about 50.0 grams (g)/L.

In some embodiments, the first glass vial comprises an aqueous solution of magnesium where the magnesium can be administered at one or more dose(s) of 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, 15.0, 15.5, 16.0, 16.5, 17.0, 17.5, 18.0, 18.5, 19.0, 19.5, 20.0, 20.5, 21.0, 21.5, 22.0, 22.5, 23.0, 23.5, 24.0, 24.5, 25.0, 25.5, 26.0, 26.5, 27.0, 27.5, 28.0, 28.5, 29.0, 29.5, 30.0, 30.5, 31.0, 31.5, 32.0, 32.5, 33.0, 33.5, 34.0, 34.5, 35.0, 35.5, 36.0, 36.5, 37.0, 37.5, 38.0, 38.5, 39.0, 39.5, 40.0, 40.5, 41.0, 41.5, 42.0, 42.5, 43.0, 43.5, 44.0, 44.5, 45.0, 45.5, 46.0, 46.5, 47.0, 47.5, 48.0, 48.5, 49.0, 49.5, 50.0, 55.0, 60.0, 65.0, 70.0, 75.0, 80.0, 85.0, 90.0, 95.0, 100.0, 105.0, 110.0, 115.0, 120.0, 125.0, 130.0, 135.0, 140.0, 145.0, 150.0, 155.0, 160.0, 165.0, 170.0, 175.0, 180.0, 185.0, 190.0, 195.0, 200.0, 200.5, 201.0, 201.5, 202.0, 202.5, 203.0, 203.5, 204.0, 204.5, 205.0, 205.5, 206.0, 206.5, 207.0, 207.5, 208.0, 208.5, 209.0, 209.5, 210.0, 210.5, 211.0, 211.5, 212.0, 212.5, 213.0, 213.5, 214.0, 214.5, 215.0, 215.5, 216.0, 216.5, 217.0, 217.5, 218.0, 218.5, 219.0, 219.5, 220.0, 220.5, 221.0, 221.5, 222.0, 222.5, 223.0, 223.5, 224.0, 224.5, 225.0, 225.5, 226.0, 226.5, 227.0, 227.5, 228.0, 228.5, 229.0, 229.5, 230.0, 230.5, 231.0, 231.5, 232.0, 232.5, 233.0, 233.5, 234.0, 234.5, 235.0, 235.5, 236.0, 236.5, 237.0, 237.5, 238.0, 238.5, 239.0, 239.5, 240.0, 240.5, 241.0, 241.5, 242.0, 242.5, 243.0, 243.5, 244.0, 244.5, 245.0, 245.5, 246.0, 246.5, 247.0, 247.5, 248.0, 248.5, 249.0, 249.5, 250.0, 255.0, 260.0, 265.0, 270.0, 275.0, 280.0, 285.0, 290.0, 295.0, 300.0, 305.0, 310.0, 315.0, 320.0, 325.0, 330.0, 335.0, 340.0, 345.0, 350.0, 355.0, 360.0, 365.0, 370.0, 375.0, 380.0, 385.0, 390.0, 395.0 or 400.0 mg/kg of patient body weight (e.g., actual body weight or ideal body weight).

In some embodiments, the first glass vial comprises an aqueous solution of magnesium where the magnesium can be administered in a dosing range from about 0.01 to about 0.5 mmol of Mg ions/kg. In some embodiments, the first glass vial comprises an aqueous solution of magnesium where the magnesium can be administered in a dose of about 0.01, 0.02, 0.03, 0.04, 0.05, 0.06, 0.07, 0.08, 0.09, 0.1, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.30, 0.31, 0.32, 0.33, 0.34, 0.35, 0.36, 0.37, 0.38, 0.39, 0.40, 0.41, 0.42, 0.43, 0.44, 0.45, 0.46, 0.47, 0.48, 0.49, 0.5, 0.6, 0.7, 0.8, 0.9, 1.0, 1.5, 2.0, 2.2, 2.4, 2.5, 2.6, 2.8, 3.0, 3.2, 3.4, 3.5, 3.8, 4.0, 4.2, 4.4, 4.5, 5.0, 5.5, 6.0, 6.5, 7.0, 7.5, 8.0, 8.5, 9.0, 9.5, 10.0, 10.5, 11.0, 11.5 mmol Mg ions/kg.

In some embodiments, the first glass vial comprises an aqueous solution of magnesium can be administered in a dosing range from about 0.02 to about 0.2 mmol of Mg ions/kg. In some embodiments, the magnesium can be administered in a dose of about 0.02, 0.03, 0.04, 0.05, 0.06, 0.07, 0.08, 0.09, 0.1, 0.1, 0.11, 0.12, 0.13, 0.14, 0.15, 0.16, 0.17, 0.18, 0.19 and/or 0.2 mmol Mg ions/kg.

In various embodiments, the second vial comprises an aqueous solution containing PEG and sodium chloride where the PEG is administered at about 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, 15.0, 15.5, 16.0, 16.5, 17.0, 17.5, 18.0, 18.5, 19.0, 19.5, 20.0, 20.5, 21.0, 21.5, 22.0, 22.5, 23.0, 23.5, 24.0, 24.5, 25.0, 25.5, 26.0, 26.5, 27.0, 27.5, 28.0, 28.5, 29.0, 29.5, 30.0, 30.5, 31.0, 31.5, 32.0, 32.5, 33.0, 33.5, 34.0, 34.5, 35.0, 35.5, 36.0, 36.5, 37.0, 37.5, 38.0, 38.5, 39.0, 39.5, 40.0, 40.5, 41.0, 41.5, 42.0, 42.5, 43.0, 43.5, 44.0, 44.5, 45.0, 45.5, 46.0, 46.5, 47.0, 47.5, 48.0, 48.5, 49.0, 49.5, 50.0, 50.5, 51.0, 51.5, 52.0, 52.5, 53.0, 53.5, 54.0, 54.5, 55.0, 55.5, 56.0, 56.5, 57.0, 57.5, 58.0, 58.5, 59.0, 59.5 to about 60% w/v, w/w and/or v/v.

In some embodiments, the second vial comprises an aqueous solution containing PEG and sodium chloride where the PEG is administered at about 0.10, 0.11, 0.12, 0.13, 0.14, 0.15, 0.16, 0.17, 0.18, 0.19, 0.20, 0.21, 0.22, 0.23, 0.24, 0.25, 0.26, 0.27, 0.28, 0.29, 0.30, 0.31, 0.32, 0.33, 0.34, 0.35, 0.36, 0.37, 0.38, 0.39, 0.40, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 071, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, 15.0, 15.5, 16.0, 16.5, 17.0, 17.5, 18.0, 18.5, 19.0, 19.5, 20.0, 20.5, 21.0, 21.5, 22.0, 22.5, 23.0, 23.5, 24.0, 24.5, 25.0, 25.5, 26.0, 26.5, 27.0, 27.5, 28.0, 28.5, 29.0, 29.5, 30.0, 30.5, 31.0, 31.5, 32.0, 32.5, 33.0, 33.5, 34.0, 34.5, 35.0, 35.5, 36.0, 36.5, 37.0, 37.5, 38.0, 38.5, 39.0, 39.5, 40.0, 40.5, 41.0, 41.5, 42.0, 42.5, 43.0, 43.5, 44.0, 44.5, 45.0, 45.5, 46.0, 46.5, 47.0, 47.5, 48.0, 48.5, 49.0, 49.5 50.0, 50.5, 51.0, 51.5, 52.0, 52.5, 53.0, 53.5, 54.0, 54.5, 55.0, 55.5, 56.0, 56.5, 57.0, 57.5, 58.0, 58.5, 59.0, 59.5 to about 60.0 grams (g)/L.

In some embodiments, the second vial comprises an aqueous solution containing PEG and sodium chloride where the PEG is administered at about one or more dose(s) of 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, 15.0, 15.5, 16.0, 16.5, 17.0, 17.5, 18.0, 18.5, 19.0, 19.5, 20.0, 20.5, 21.0, 21.5, 22.0, 22.5, 23.0, 23.5, 24.0, 24.5, 25.0, 25.5, 26.0, 26.5, 27.0, 27.5, 28.0, 28.5, 29.0, 29.5, 30.0, 30.5, 31.0, 31.5, 32.0, 32.5, 33.0, 33.5, 34.0, 34.5, 35.0, 35.5, 36.0, 36.5, 37.0, 37.5, 38.0, 38.5, 39.0, 39.5, 40.0, 40.5, 41.0, 41.5, 42.0, 42.5, 43.0, 43.5, 44.0, 44.5, 45.0, 45.5, 46.0, 46.5, 47.0, 47.5, 48.0, 48.5, 49.0, 49.5, 50.0, 55.0, 60.0, 65.0, 70.0, 75.0, 80.0, 85.0, 90.0, 95.0, 100.0, 105.0, 110.0, 115.0, 120.0, 125.0, 130.0, 135.0, 140.0, 145.0, 150.0, 155.0, 160.0, 165.0, 170.0, 175.0, 180.0, 185.0, 190.0, 195.0, 200.0, 200.5, 201.0, 201.5, 202.0, 202.5, 203.0, 203.5, 204.0, 204.5, 205.0, 205.5, 206.0, 206.5, 207.0, 207.5, 208.0, 208.5, 209.0, 209.5, 210.0, 210.5, 211.0, 211.5, 212.0, 212.5, 213.0, 213.5, 214.0, 214.5, 215.0, 215.5, 216.0, 216.5, 217.0, 217.5, 218.0, 218.5, 219.0, 219.5, 220.0, 220.5, 221.0, 221.5, 222.0, 222.5, 223.0, 223.5, 224.0, 224.5, 225.0, 225.5, 226.0, 226.5, 227.0, 227.5, 228.0, 228.5, 229.0, 229.5, 230.0, 230.5, 231.0, 231.5, 232.0, 232.5, 233.0, 233.5, 234.0, 234.5, 235.0, 235.5, 236.0, 236.5, 237.0, 237.5, 238.0, 238.5, 239.0, 239.5, 240.0, 240.5, 241.0, 241.5, 242.0, 242.5, 243.0, 243.5, 244.0, 244.5, 245.0, 245.5, 246.0, 246.5, 247.0, 247.5, 248.0, 248.5, 249.0, 249.5, 250.0, 255.0, 260.0, 265.0, 270.0, 275.0, 280.0, 285.0, 290.0, 295.0, 300.0, 300.5, 301.0, 301.5, 302.0, 302.5, 303.0, 303.5, 304.0, 304.5, 305.0, 305.5, 306.0, 306.5, 307.0, 307.5, 308.0, 308.5, 309.0, 309.5, 310.0, 310.5, 311.0, 311.5, 312.0, 312.5, 313.0, 313.5, 314.0, 314.5, 315.0, 316.0, 316.5, 317.0, 317.5, 318.0, 318.5, 319.0, 319.5, 320.0, 320.5, 321.0, 321.5, 322.0, 322.5, 323.0, 323.5, 324.0, 324.5, 325.0, 325.5, 326.0, 326.5, 327.0, 327.5, 328.0, 328.5, 329.0, 329.5, 330.0, 330.5, 331.0, 331.5, 332.0, 332.5, 333.0, 333.5, 334.0, 334.5, 335.0, 335.5, 336.0, 336.5, 337.0, 337.5, 338.0, 338.5, 339.0, 339.5, 340.0, 340.5, 341.0, 341.5, 342.0, 342.5, 343.0, 343.5, 344.0, 344.5, 345.0, 345.5, 346.0, 346.5, 347.0, 347.5, 348.0, 348.5, 349.0, 349.5, 350.0, 355.0, 360.0, 365.0, 370.0, 375.0, 380.0, 385.0, 390.0, 395.0 or 400.0 mg/kg of patient body weight (e.g., actual body weight or ideal body weight).

In some embodiments, the compositions of the present application may be provided in one or more vials, ampules, prefilled syringes, bottles, bags, and/or other containers. In some embodiments, the compositions, vials, ampules, prefilled syringes, bottles, bags, and/or other containers can be sterilized and/or preservative free. These may be single or multidose vials, ampules, prefilled syringes, bottles, bags, and/or other containers. These vials, ampules, prefilled syringes, bottles, bags, and/or other containers can be made from inert material (e.g., glass, polytetrafluoroethyleneteflon, or the like) and/or be colored (e.g., amber) to protect the compositions from light to improve shelf-life stability or the container can be colorless.

For example, the PEG (10%-60%) and magnesium (0.01%-20%) can be provided in a kit in sterile separate vials and then each mixed separately or together to a larger volume with one or more suitable diluents (e.g., D5W, NS, 0.45% saline, SWFI etc.) to further dilute the compositions for ease of delivery. There may also be provided other sterile empty single or multidose vials, ampules, syringes, bottles, bags, and/or other containers for ease of mixing and adminsitering. The kit may also contain needles, transfer units, sterile alcohol swabs for ease of mixing and administration.

In various embodiments, the composition comprises magnesium chloride, polyethylene glycol, sodium chloride (NaCl) and/or sterile water for injection (SWFI). In some embodiments, the composition comprises additional diluents such as, for example, D5W, D5NS and D5W/1/2NS.

In some embodiments, the composition comprising PEG and magnesium that are in the same composition can be administered by or within 6 hours after the injury (e.g., spinal cord, traumatic brain injury, stroke). In some embodiments, the composition comprising PEG and magnesium can be administered by or within 5 hours after the injury (e.g., spinal cord, traumatic brain injury, stroke). In some embodiments, the composition comprising PEG and magnesium can be administered by or within 4 hours after the injury (e.g., spinal cord, traumatic brain injury, stroke). In some embodiments, the composition comprising PEG and magnesium can be administered by or within 3 hours after the injury (e.g., spinal cord, traumatic brain injury, stroke). In some embodiments, the composition comprising PEG and magnesium can be administered by or within 2 hours after the injury (e.g., spinal cord, traumatic brain injury, stroke). In some embodiments, the composition comprising PEG and magnesium can be administered by or within 1 hour after the injury (e.g., spinal cord, traumatic brain injury, stroke). In some embodiments, the composition comprising PEG and magnesium can be administered by or within 30 minutes after the injury (e.g., spinal cord, traumatic brain injury, stroke). In some embodiments, the composition comprising PEG and magnesium can be administered by or within 20 minutes after the injury (e.g., spinal cord, traumatic brain injury). In some embodiments, the composition comprising PEG and magnesium can be administered by or within 15 minutes after the injury (e.g., spinal cord, traumatic brain injury, stroke). In some embodiments, the composition comprising PEG and magnesium can be administered by or within 10 minutes after the injury (e.g., spinal cord, traumatic brain injury, stroke). In some embodiments, the composition comprising PEG and magnesium can be administered by or within 5 minutes after the injury (e.g., spinal cord, traumatic brain injury, stroke) or as close as possible to the injury.

In some embodiments, the composition comprising PEG can be administered by or within 6 hours after the injury (e.g., spinal cord, traumatic brain injury). In some embodiments, the composition comprising PEG can be administered by or within 5 hours after the injury (e.g., spinal cord, traumatic brain injury). In some embodiments, the composition comprising PEG can be administered by or within 4 hours after the injury (e.g., spinal cord, traumatic brain injury). In some embodiments, the composition comprising PEG can be administered by or within 3 hours after the injury (e.g., spinal cord, traumatic brain injury). In some embodiments, the composition comprising PEG can be administered by or within 2 hours after the injury (e.g., spinal cord, traumatic brain injury). In some embodiments, the composition comprising PEG can be administered by or within 1 hour after the injury (e.g., spinal cord, traumatic brain injury). In some embodiments, the composition comprising PEG can be administered by or within 30 minutes after the injury (e.g., spinal cord, traumatic brain injury). In some embodiments, the composition comprising PEG can be administered by or within 20 minutes after the injury (e.g., spinal cord, traumatic brain injury). In some embodiments, the composition comprising PEG can be administered by or within 15 minutes after the injury (e.g., spinal cord, traumatic brain injury). In some embodiments, the composition comprising PEG can be administered by or within 10 minutes after the injury (e.g., spinal cord, traumatic brain injury). In some embodiments, the composition comprising PEG can be administered by or within 5 minutes after the injury (e.g., spinal cord, traumatic brain injury) or as close as possible to the injury.

In some embodiments, the composition comprising magnesium can be administered by or within 12 hours after the injury (e.g., spinal cord, traumatic brain injury). In some embodiments, the composition comprising magnesium can be administered by or within 11 hours after the injury (e.g., spinal cord, traumatic brain injury). In some embodiments, the composition comprising magnesium can be administered by or within 10 hours after the injury (e.g., spinal cord, traumatic brain injury). In some embodiments, the composition comprising magnesium can be administered by or within 9 hours after the injury (e.g., spinal cord, traumatic brain injury). In some embodiments, the composition comprising magnesium can be administered by or within 8 hours after the injury (e.g., spinal cord, traumatic brain injury). In some embodiments, the composition comprising magnesium can be administered by or within 7 hours after the injury (e.g., spinal cord, traumatic brain injury). In some embodiments, the composition comprising magnesium can be administered by or within 6 hours after the injury (e.g., spinal cord, traumatic brain injury). In some embodiments, the composition comprising magnesium can be administered by or within 5 hours after the injury (e.g., spinal cord, traumatic brain injury). In some embodiments, the composition comprising magnesium can be administered by or within 4 hours after the injury (e.g., spinal cord, traumatic brain injury). In some embodiments, the composition comprising magnesium can be administered by or within 3 hours after the injury (e.g., spinal cord, traumatic brain injury). In some embodiments, the composition comprising magnesium can be administered by or within 2 hours after the injury (e.g., spinal cord, traumatic brain injury). In some embodiments, the composition comprising magnesium can be administered by or within 1 hour after the injury (e.g., spinal cord, traumatic brain injury). In some embodiments, the composition comprising magnesium can be administered by or within 30 minutes after the injury (e.g., spinal cord, traumatic brain injury). In some embodiments, the composition comprising magnesium can be administered by or within 20 minutes after the injury (e.g., spinal cord, traumatic brain injury). In some embodiments, the composition comprising magnesium can be administered by or within 15 minutes after the injury (e.g., spinal cord, traumatic brain injury). In various embodiments, the composition comprising magnesium can be administered by or within 10 minutes after the injury (e.g., spinal cord, traumatic brain injury). In some embodiments, the composition comprising magnesium can be administered by or within 5 minutes after the injury (e.g., spinal cord, traumatic brain injury) or as close as possible to the injury.

In various embodiments, the composition comprising magnesium and the composition comprising PEG are administered concurrently by or within 6 hours after the injury (e.g., spinal cord, traumatic brain injury). In some embodiments, the composition comprising magnesium and the composition comprising PEG are administered concurrently by or within 5 hours after the injury (e.g., spinal cord, traumatic brain injury). In some embodiments, the composition comprising magnesium and the composition comprising PEG are administered concurrently by or within 4 hours after the injury (e.g., spinal cord, traumatic brain injury). In some embodiments, the composition comprising magnesium and the composition comprising PEG are administered concurrently by or within 3 hours after the injury (e.g., spinal cord, traumatic brain injury). In some embodiments, the composition comprising magnesium and the composition comprising PEG are administered concurrently by or within 2 hours after the injury (e.g., spinal cord, traumatic brain injury). In some embodiments, the composition comprising magnesium and the composition comprising PEG are administered concurrently by or within 1 hour after the injury (e.g., spinal cord, traumatic brain injury). In some embodiments, the composition comprising magnesium and the composition comprising PEG are administered concurrently by or within 30 minutes after the injury (e.g., spinal cord, traumatic brain injury). In some embodiments, the composition comprising magnesium and the composition comprising PEG are administered concurrently by or within 20 minutes after the injury (e.g., spinal cord, traumatic brain injury). In some embodiments, the composition comprising magnesium and the composition comprising PEG are administered concurrently by or within 15 minutes after the injury (e.g., spinal cord, traumatic brain injury). In some embodiments, the composition comprising magnesium and the composition comprising PEG are administered concurrently by or within 10 minutes after the injury (e.g., spinal cord, traumatic brain injury). In some embodiments, the composition comprising magnesium and the composition comprising PEG are administered concurrently by or within 5 minutes after the injury (e.g., spinal cord, traumatic brain injury) or as close as possible to the injury.

In some embodiments, one or more doses of magnesium and PEG where the dose of magnesium was about 3.9, 7.8, 15.6, 23.4 and/or 39 mg/kg achieved magnesium serum levels having a Cₘᵢₙ to Cₘₐₓ of about 0.75 mmol/L to 0.9 mmol/L at 5 minutes; about 1.2 mmol/L to 1.6 mmol/L at 0.25 hours; about 1.55 mmol/L to 1.75 mmol/L at 0.5 hours; about 1.4 mmol/L to 1.6 mmol/L at 1 hour; about 1.2 mmol/L to 1.5 mmol/L at 2 hours; about 1.1 mmol/L to 1.35 mmol/L at 3 hours; about 1.1 mmol/L to 1.3 mmol/L at 4 hours and about 1.05 mmol/L to 1.2 mmol/L at 5 hours.

In some embodiments, one or more doses of magnesium and PEG where the dose of magnesium was about 3.9, 7.8, 15.6, 23.4 and/or 39 mg/kg achieved magnesium serum levels having a Cₘᵢₙ of about 0.75 mmol/L at 5 minutes; a Cₘᵢₙ of about 1.2 mmol/L at 0.25 hours; a Cₘᵢₙ of about 1.55 mmol/L at 0.5 hours; a Cₘᵢₙ of about 1.4 mmol/L at 1 hour; a Cₘᵢₙ of about 1.2 mmol/L at 2 hours; a Cₘᵢₙ of about 1.1 mmol/L at 3 hours; a Cₘᵢₙ of about 1.1 mmol/L at 4 hours; and/or a Cₘᵢₙ of about 1.05 mmol/L at 5 hours.

In some embodiments, one or more doses of magnesium and PEG where the dose of magnesium was about 3.9, 7.8, 15.6, 23.4 and/or 39 mg/kg achieved magnesium serum levels having a Cₘᵢₙ to Cₘₐₓ of about 0.75 mmol/L to 0.9 mmol/L; about 1.2 mmol/L to 1.6 mmol/L; about 1.55 mmol/L to 1.75 mmol/L; about 1.4 mmol/L to 1.6 mmol/L; about 1.2 mmol/L to 1.5 mmol/L; about 1.1 mmol/L to 1.35 mmol/L; about 1.1 mmol/L to 1.3 mmol/L and about 1.05 mmol/L to 1.2 mmol/L.

In some embodiments, one or more doses of magnesium and PEG where the dose of magnesium was about 3.9, 7.8, 15.6, 23.4 and/or 39 mg/kg achieved magnesium serum levels having a Cₘᵢₙ of about 0.75 mmol/L; a Cₘᵢₙ of about 1.2 mmol/L; a Cₘᵢₙ of about 1.55 mmol/L; a Cₘᵢₙ of about 1.4 mmol/L; a Cₘᵢₙ of about 1.2 mmol/L; a Cₘᵢₙ of about 1.1 mmol/L; a Cₘᵢₙ of about 1.1 mmol/L; and/or a Cₘᵢₙ of about 1.05 mmol/L.

In some embodiments, the compositions comprising magnesium and/or PEG can be given in 1, 2, 3, 4, 5, 6, 7, 8, 9, 10, 11, 12, 13, 14, 15, 16, 17, 18, 19, 20, 21, 22, 23 or 24 hour intervals.

In some embodiments, the patient receives the PEG and/or magnesium by or within six hours of the injury (e.g., spinal cord and/or traumatic brain injury) and then 5 additional doses of the PEG and/or magnesium by or within 30 hours of the injury.

In some embodiments, the PEG and/or the magnesium is used to treat spinal cord injury.

In some embodiments, the PEG and/or the magnesium is used to treat ischemic injury.

In some embodiments, the PEG and/or the magnesium is used to treat hemorrhagic stroke injury.

In some embodiments, the PEG and/or the magnesium is used to treat brain injury.

In some embodiments, the PEG and/or the magnesium is used to treat traumatic brain injury.

In some embodiments, the PEG and/or the magnesium is used to treat nerve injury.

In some embodiments, the PEG and/or the magnesium is used to treat peripheral nerve injury.

In some embodiments, the methods and compositions provided may: (i) consist of only the PEG, magnesium and the delivery vehicle; or (ii) consist essentially of the PEG, magnesium and the delivery vehicle; or (iii) comprise the PEG, magnesium, an antioxidant and the delivery vehicle or other ingredients or combinations thereof.

Examples of suitable pathological conditions that the compositions and methods can be used to treat include, metabolic neuropathies such as diabetic and alcoholic neuropathies, postherpetic neuralgia, trauma to the central nervous system such as stroke, traumatic brain, spinal cord (e.g., acute spinal cord injury) or cauda equine injuries. In some embodiments, PEG and the magnesium may be delivered independently of each other by a bolus infusion, a continuous infusion, an intravenous administration (IV), an intramuscular administration, intrathecal administration, subcutaneous administration, epidural administration, intra-articular administration, parenteral administration, direct application onto or adjacent to a site of the pathological condition, and any combinations thereof. Initiation of individual treatments could be separated by a few hours, e.g., up to about 24 hours, or more preferably, up to about 16 hours, or more preferably of up to about 8 hours, or even more preferably, up to 6 hours. Thus, PEG and the magnesium may be delivered from independent sources and/or by different methods, or they may be mixed prior to delivery in the same or separate compositions.

A person of ordinary skill in the art will further recognize that certain invasive procedures, such as, for example, brain or spinal cord surgeries, leave a subject with a neuronal injury. Accordingly, in one embodiment of the disclosure, PEG and/or magnesium is delivered to the subject prior to the event triggering the occurrence of the pathological condition. In one example, the event is brain surgery and the pathological condition is an injury to CNS neurons.

In various embodiments, parameters are monitored in the following studies by taking baseline serum levels of biomarkers before administering the PEG and/or magnesium. The biomarkers and serum and CSF magnesium and/or PEG are then measured during and/or after treatment. In some embodiments, suitable biomarkers include ions, amino acids, neurotransmitters, sugars, lipids, carbohydrates, proteins, peptides, enzymes, receptors, hormones, steroids, genes, ribonucleotides, etc. or any combination thereof. Other more specific biomarkers include magnesium, calcium, glutamate, glutamine, choline, acetylcholine esterase, tau, c-tau, neuron-specific enolase, ubiquitin and ubiquitin enzymes such as ubiquitin hydrolases, n-acetylaspartate, neuronal filaments, myo-inositol, S-100β, interleukins, antibodies against the polymer such as antibodies against PEG as described in U.S. Application Serial No. 12/105,666 filed April 18, 2008, published as U.S. Application Publication No. 2009/0263507. In various embodiments, the biomarkers comprise cytokines, such as, for example IL-6, IL-8, MCP-1, tau, and glial fibrillary acidic protein (GFAP). (Kwon et al. Journal of Neurotrauma 27:669-682 April 2010).

In some embodiments, biomarkers are taken before, during and/or after injury to a patient. In various embodiments, the biomarkers include a cleaved form of MAP-tau (c-tau), phosphorylated axonal form of neurofilament subunit NF-H (pNF-H), ubiquitin C-terminal hydrolase 1 (UCHL1), S-100β, neuron specific enolase (NSE) and IL-6. In some embodiments, the biomarker levels increase following injury (e.g., spinal cord injury and traumatic brain injury) and/or the biomarker levels decrease when PEG and/or magnesium is administered the site of injury. In some embodiments, the biomarker comprises pNF-H and levels of pNF-H increase following injury (e.g., spinal cord injury and traumatic brain injury) and/or pNF-H levels decrease when PEG and/or magnesium is administered to the patient.

Additional features and advantages of various embodiments will be set forth in part in the description that follows, and in part will be apparent from the description, or may be learned by practice of various embodiments. The objectives and other advantages of various embodiments will be realized and attained by means of the elements and combinations particularly pointed out in the description and appended claims.

### EXAMPLES

### Example 1

### PEG and Magnesium in Rats

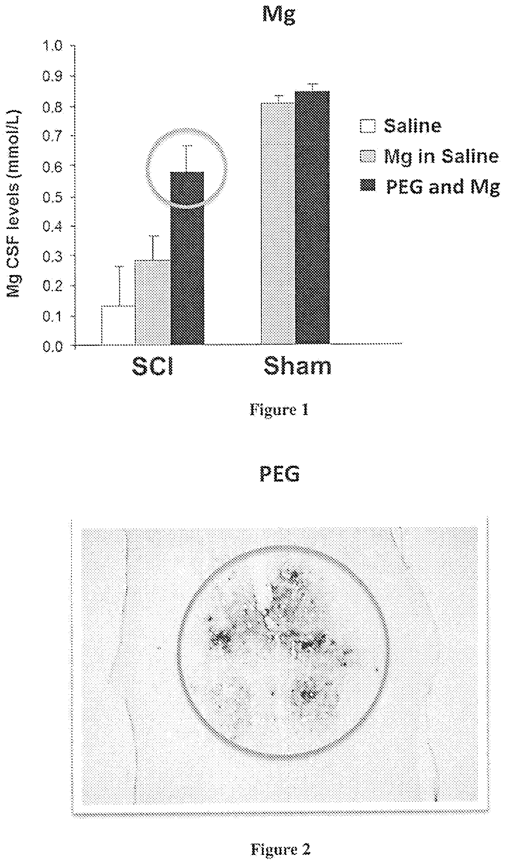

Figure 1 depicts a graph showing preferential distribution of magnesium into cerebrospinal fluid (CSF) following parenteral administration of magnesium in a PEG formulation relative to magnesium in saline formulation and a saline formulation initiated a few hours after injury in rats. Two groups were administered the three formulations. Group 1 comprised animals with spinal cord injury and Group 2 comprised animals that were not injured. Group 1 results showed that saline had about 0.13 mmol/L, magnesium in saline had about 0.28 mmol/L, and PEG/magnesium had about 0.58 mmol/L of magnesium CSF levels. Group 2 results showed that magnesium in saline had about 0.8 mmol/L, and PEG/magnesium had about 0.84 mmol/L of magnesium CSF levels. The graph results reflect that the combination of PEG and magnesium has a higher distribution of magnesium than both the magnesium in saline formulation and saline formulation. When injury occurs, electrolyte levels decrease (i.e., magnesium), causing neurons to die. PEG unexpectedly enhanced magnesium accumulation in the CSF thereby facilitating neuron survival. When magnesium accumulates, the electrolytes reach homeostasis thereby preventing neuron death. The results were described in U.S. Application Serial No. 12/411,572 filed March 26, 2009, published as U.S. Application Publication No. 2010/0247677, in which the entire disclosure is incorporated by reference

### Example 2

Male Sprague-Dawley Rats were anesthetized and placed in a prone position on a stereotaxic frame. A T9/10 laminectomy was performed and animals were contused at a displacement of 1.5 mm with the Ohio State University impactor.

Soon after injury, saline, 0.8% magnesium in saline, 0.8% magnesium in 20% or 30% PEG3350, PEG-biotin or magnesium in a PEG3000-biotin formulations were administered by intravenous infusion of 5-7 mL/kg over a 10-30-min period. Animals received 1 to 5 infusions with an interval of 6 hours between infusions. The contents of the infusion vials were blinded to the investigators performing both the infusions and the analyses.

At various time points after infusion, the tissue we extracted and processed for histological evaluation (FIG. 2), bioanalytical analysis or functional analysis.

For the histology analysis presented at FIG. 2, the cords were harvested at the indicated timepoints, cut horizontally at 20 um thickness and processed using the colorimetric ABC kit that includes reaction with avidin-peroxidase complexes and the peroxidase susbrate DAB leading to the development of a brown color where the biotin molecules (here the PEG-biotin) are located.

For the bioanalytical analysis, tissue containing or not the site of hemorrhage were collected. Tissue concentrations of PEG in rat plasma were measured using a validated high performance liquid chromatograpy tandem mass spectrometry (HPLC/MS/MS) method in positive electrospray ionization mode. Following centrifugation of the tissue homogenate, the supernatant fraction from each sample was concentrated by evaporation and reconstituted with mobile phase A prior to analysis. The samples were analyzed with an HPLC/MS/MS assay using a Thermo Hypersil ODS column. The peak areas of PEG and the theoretical concentrations of calibration standards were fit to the In-quadratic function, excluding the origin.

The following method describes the functional analysis or evaluation of the extent of hemorrhage at the injury site. At 72 hours post-injury, the animals were decapitated and a 15 mm segment of the spinal cord centered around the hemorrhagic site was collected and frozen and cyrosectioned at a thickness of 20 um. Eleven sections per cord were selected for analysis: the epicentre of injury, and the sections rostral and caudal 400 um, 800 um, 1600 um, 2800 um, and 3200 um. The slides were cover slipped and color images of the spinal cords were obtained at 5× objective using a Leica light microscope. The red channel (representing bleeding into the tissue) was captured on a greyscale image, and the intensity threshold was set at 230 across all images to remain consistent throughout. The spinal cords, excluding the dura and blood trapped underneath, were circled in green. The overlapping signals of expressed red and circled green was highlighted with blue using the Overlay Math function under Image from the toolbar in Sigma Scan and the total area of blue signal was measured as the extent of hemorrhage.

### Results

(1) PEG accumulates and is retained preferentially at the hemorrhagic site following a single parenteral administration.
   Referring to FIG. 2, histological DAB staining following parenteral administration of the biotin-labeled PEG solution indicated that the biotin-labeled PEG accumulates preferentially at the hemorrhagic site with no visible accumulation in the spinal cord regions above or below the site of injury. PEG could be detected at the hemorrhagic site within 2 hours after administration, the PEG signal increased over 24 hours after administration and had largely disappeared by 7 days post-administration. Non-specific DAB staining was absent in the spinal cords of saline-treated animals.
(2) PEG accumulation at the hemorrhagic site is influenced by the mode of administration.
   Histological DAB staining indicated that significantly higher accumulation and retention of the biotin-labeled PEG was achieved 24 hours following a single intravenous injection relative to a single intraperitoneal injection of the biotin-labeled PEG solution. However, there was no obvious difference in the biotin-labeled PEG signal found at the hemorrhagic site 2 hours following intravenous injection or intraperitoneal injection of the biotin-labeled PEG solution.
(3) Compositions of 20% and 30% of PEG solution lead to similar accumulations of PEG at the hemorrhagic site after a single or repeated intravenous infusions.
   Quantitative evaluation of PEG spinal tissue levels using HPLC/MS/MS assay indicated that PEG accumulates preferentially at the hemorrhagic site following one intravenous infusions of a magnesium in 20% PEG3350 solution with PEG tissue levels of 936 ng/ml found at the hemorrhagic site relative to 315 ng/ml at a non-injured site at 3 hours post-infusion. Following five infusions of magnesium in 20% PEG3350 solution with an interval of 6 hours between each infusion, the PEG tissue levels reached 4025 ng/ml at the hemorrhagic site relative to 595 ng/ml at a non-injured site 3 hours after the last infusion. Similarly, following a single intravenous administration of magnesium in 30% PEG3350 solution, PEG tissue levels of 1430 ng/ml were found at the hemorrhagic site relative to 519 ng/ml at a non-injured site at 3 hours post-infusion. Following five infusions of magnesium in 30% PEG3350 solution with an interval of 6 hours between each infusion, the PEG tissue levels reached 3891 ng/ml at the hemorrhagic site relative to 526 ng/ml at a non-injured site 3 hours after the last infusion,
   Intravenous administration of PEG reduces the extent of the hemorrhage at the site of injury. Addition of magnesium to the PEG formulation further decreases the hemorrhagic signal.
   Quantitative morphometric analysis of the hemorrhagic signal within an area covering 3.2 mm² of the epicenter of the injury site. Saline treatment following SCI in rats led to bleeding detected in an area size of 0.79 mm². The extent of the bleeding area was reduced to 0,47 mm² following intravenous administration of a PEG solution and further reduced to 0.25 mm² following intravenous administration of a magnesium in PEG solution. (U.S. Application Serial No. 12/411,548 filed March 26, 2009, published as U.S. Application Publication No. 2010/0247676, in which the entire disclosure is incorporated by reference).

### Example 3

In addition to the rat data in Examples 1 and 2, a study evaluated safety, tolerability and pharmacokinetics of intravenously administered PEG and magnesium in 70 healthy human volunteers. This study also collected comparative data with MgSO4. It was designed as a single-center, single and multiple infusion(s), double-blind, randomized study comparing PEG and magnesium to a placebo (0.45% normal saline) and to MgSO4 solution for injection.

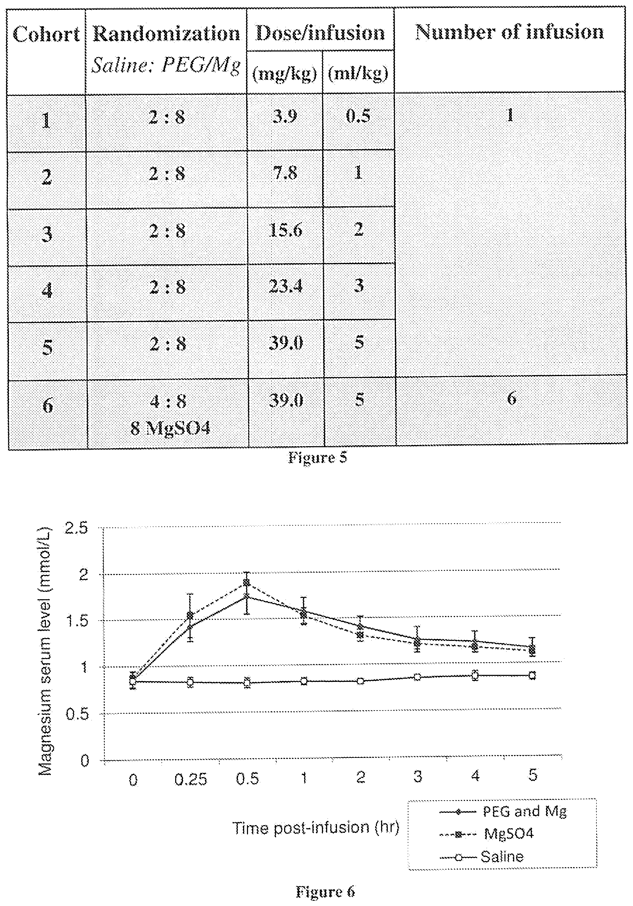

The study had Study Arm A and Study Arm B. Study Arm A was composed of 5 cohorts and 50 subjects. In each cohort, 8 subjects received PEG and magnesium and 2 received a placebo. The dose-escalation paradigm was 3.9, 7.8, 15.6, 23.4 and 39 mg/kg corresponding to 0.5, 1, 2, 3 and 5 mL/kg of PEG and magnesium and compared to equivalent volumes of placebo (Fig. 5). All subjects received a single infusion delivered over approximately 30 minutes. Safety was assessed before continuing to the next cohort. Upon completion of Study Arm A, the maximum tolerated dose (MTD) within the range tested was established and utilized in Study Arm B.

Study Arm B was composed of 8 subjects who received 6 infusions of 39 mg/kg/infusion of PEG and magnesium for a total dose of 234 mg/kg, 8 subjects who received 6 infusions of an equivalent magnesium dose of MgSO4 for injection and 4 subjects who received the equivalent volume of placebo. Each infusion was delivered over 30 min and there was a 6-hour interval (from the beginning of one infusion to the beginning of the next infusion) between infusions.

Figure 6 provides a graph showing magnesium serum levels in mmol/L during different post-infusion time periods following parenteral administration of magnesium in a PEG formulation relative to a MgSO4 formulation and a saline formulation for the doses in Figure 5. The results of the graph show that the combination of PEG and magnesium lead to similar Mg systemic exposure when compared to MgSO4. Magnesium serum levels correlating to the combination of a PEG and magnesium formulation were Cₘᵢₙ = about 0.75 mmol/L, C_{median} = about 0.85 mmol/L, Cₘₐₓ = about 0.9 mmol/L at 5 minutes; Cₘᵢₙ = about 1.2 mmol/L, C_{median} = about 1.4 mmol/L, Cₘₐₓ = about 1.6 mmol/L at 0.25 hours; Cₘᵢₙ = about 1.55 mmol/L, C_{median} = about 1.7 mmol/L, Cₘₐₓ = about 1.75 mmol/L at 0.5 hours; Cₘᵢₙ = about 1.4 mmol/L, C_{median} = about 1.5 mmol/L, Cₘₐₓ = about 1.6 mmol/L at 1 hour; Cₘᵢₙ = about 1.2 mmol/L, C_{median} = about 1.35 mmol/L, Cₘₐₓ = about 1.5 mmol/L at 2 hours; Cₘᵢₙ = about 1.1 mmol/L, C_{median} = about 1.25 mmol/L, Cₘₐₓ = about 1.35 mmol/L at 3 hours; Cₘᵢₙ = about 1.1 mmol/L, C_{median} = about 1.2 mmol/L, Cₘₐₓ = about 1.3 mmol/L at 4 hours; and Cₘᵢₙ = about 1.05 mmol/L, C_{median} = about 1.15 mmol/L, Cₘₐₓ = about 1.2 mmol/L at 5 hours.

The results of this study showed that PEG and magnesium was well tolerated at all doses in healthy subjects and administration of PEG and magnesium or an equivalent magnesium dose of MgSO4 for injection lead to systemic exposure proportionate to total dose. All clearance and safety and tolerability profiles were similar.

The following conclusions reached based on this study were that no notable trends in safety-related issues were seen with patients who were treated with PEG and magnesium at any of the doses analyzed in this study. When compared with placebo or magnesium sulfate, there were no obvious differences in safety findings, in types of reported treatment-emergent adverse events, vital signs, or clinical laboratory findings, including electrocardiograms.

The objectives of this study were met in that a maximum dose of PEG and magnesium was well tolerated.

### Example 4

The protocol for human patients is described below for the treatment of acute spinal cord injury using injectable magnesium and polyethyleneglycol (PEG).

### Inclusion criteria

In patients to be treated, a neurological exam establishing the level and severity of injury on the American Spinal Injury Association (ASIA) impairment scale (AIS) will be employed for patient monitoring and for analysis of patient improvement. Diagnostic techniques employed during the neurological examination include but are not limited to X-rays, computed tomography (CT) scans, transcranial magnetic stimulation (TMS) and magnetic resonance imaging (MRI).

The patient having the spinal cord injury is from about 16 years to 65 years of age or 18 years to 65 years of age and is a male or a female and can receive magnesium and PEG. The patient having the spinal cord injury will be suffering from acute traumatic spinal cord injury, at neurological levels between cervical 4 vertebrae and thoracic 11 vertebrae and can receive magnesium and PEG. The patient will have an ASIA Impairment Scale (AIS) grade A, B or C and can receive magnesium and PEG. The patient with have an acute spinal cord injury with no evidence of penetrating or transection injury (e.g. caused by projectile or stab wound) and can receive magnesium and PEG.

The patient that can receive the magnesium and PEG can have one or more of the following attributes: (i) the patient will be 16 years to 65 years of age, or 18 years to 65 years of age; (ii) the patient will be suffering from acute traumatic spinal cord injury at any vertebrae; (iii) the patient will have acute traumatic spinal cord injury between cervical 4 vertebrae and thoracic 11 vertebrae; (iv) the patient will have an ASIA Impairment Scale (AIS) grade A, B or C; and/or (v) the patient with have an acute spinal cord injury with no evidence of penetrating or transection injury (e.g. caused by projectile or stab wound) and can receive magnesium and PEG.

### Exclusion criteria

The patient that will be excluded from receiving the magnesium and the PEG will have one or more of the following attributes: (i) known allergy or hypersensitivity to polyethylene glycol; (ii) mental impairment or other conditions that would preclude a reliable ASIA exam; and /or (iii) history or active renal failure or dialysis.

### Monitoring Parameters

Parameters are monitored in the following studies by taking baseline serum levels of biomarkers before administering the PEG and/or magnesium. The biomarkers and serum and CSF magnesium and/or PEG are then measured before, during and/or after treatment. Suitable biomarkers include magnesium, calcium, glutamate, glutamine, choline, acetylcholine esterase, tau, neuron-specific enolase, ubiquitin and ubiquitin hydrolases, n-acetylaspartate, neuronal filaments, myo-inositol, interleukins, antioxidants, antibodies against the polymer such as antibodies against PEG. In some embodiments, the biomarker is at least one of an ion, an amino acid, a sugar, a lipid, a protein, a peptide, receptor, neurotransmitter, enzyme, a gene or a ribonucleotide. Other more specific biomarkers include magnesium, calcium, glutamate, glutamine, choline, acetylcholine esterase, tau, c-tau, neuron-specific enolase, ubiquitin and ubiquitin enzymes such as ubiquitin hydrolases, n-acetylaspartate, neuronal filaments, myo-inositol, interleukins, antibodies against the polymer such as antibodies against PEG as described in U.S. Application Serial No. 12/105,666 filed April 18, 2008, published as U.S. Application Publication No. 2009/0263507. In some embodiments, the biomarkers comprise cytokines, such as, for example IL-6, IL-8, MCP-1, tau, S100β, and glial fibrillary acidic protein (GFAP). (Kwon et al. 2010). These entire disclosures are herein incorporated by reference into the present disclosure.

### Example 5

It is contemplated to have a double-blind, placebo-controlled study to determine the safety, tolerability and potential activity of PEG and magnesium following a regimen of 6 doses over 30 hours in patients with acute SCI. Forty (40) patients are planned for enrollment into one of the two treatment groups, PEG and magnesium or placebo, in a randomized 1:1 allocation.

The effective treatment window for PEG and magnesium in human subjects is not known, but non-clinical studies indicate that earlier treatment is more likely to result in a larger positive effect on outcome. For this initial study in patients, treatment will be initiated within 12 hours of injury.

The study period will include an acute 72-hour period of intensive safety monitoring as well as follow-up visits at 1 month, 3 months, and 6 months post-treatment to evaluate safety and efficacy outcomes. The proposed 72-hour period of intensive safety monitoring will cover the 30-hour PEG and magnesium treatment and at least 48 hours after the end of the last infusion, compared to the 6-8 hour half-life of the investigational product at steady state. During the Example 3 study, PEG and magnesium and the MgSO4 showed similar pharmacokinetic parameters with an average magnesium serum half-life of 3.7-4 hour after one infusion and 5.6-7.6 hour after six infusions. These were similar to previously published serum magnesium half-lives in healthy volunteers (i.e., 1.25-4.75 hour after one infusion) and patients (i.e., 5.2 hour for pre-eclampsia); and estimated at 6 hour in brain injured patients after a 24-hour infusion period). The 6 month follow-up is designed to examine the long-term outcome from injury later in the processes of normal neurological recovery, and will provide information relevant to potential efficacy and safety of the intervention.

As soon as possible after their arrival at the center, a full neurological examination to establish the level and severity of injury on the American Spinal Injury Association (ASIA) Impairment Scale (AIS) will be performed at the time of presentation; unless the treating physician is assured that an exam performed prior to consent is adequate according to the study guidelines. Patients with AIS grade of A, B, or C may be enrolled. Results from blood chemistry, hematology and urinalysis will be reviewed. A serum pregnancy test will be performed for women of childbearing potential. All eligibility criteria will be assessed and qualified patients will be enrolled into the study and dosed beginning no later than 12 hours post-injury with the first 30-minute intravenous (IV) infusion of PEG and magnesium or placebo. Every effort should be made to begin treatment as soon as possible, with the understanding that treatment at close to 12 hours post-injury is allowed by the protocol but likely to be sub-optimal for achieving any benefit. Patients will receive 5 additional doses of their assigned study treatment at intervals of 6 hours (a total of 6 infusions) of the PEG and magnesium.

The dosage regimen of PEG and magnesium will be 6 doses delivered at 6-hour intervals, with each dose delivered over approximately 30 min via a pump-controlled infusion.

Standard safety monitoring in trauma centers includes monitoring of vital signs, ECGs, comprehensive examinations assessing neurological functions, renal output and general physical status, evaluation of clinical chemistry/hematology soon after the injury and then at least on a daily basis.

Blood will be drawn for measurement of Mg2+ and PEG 3350 before the start of the first and last infusion, and at repeated time points after the start of the last infusion.

Published data have demonstrated that a small set of biomarkers collected from cerebral spinal fluid (CSF) and blood have positive predictive value for the eventual ASIA classification of the injury. This study will include the collection of blood samples to quantify these biomarkers and evaluate their potential to assess the pre-treatment severity of injury and provide potential measures for neuroprotective activity of the treatment. If these biomarkers appear promising for this purpose, the methods will be further validated in future clinical trials.

AIS grade will be assessed prior to dosing and repeated at specified time points after treatment. The assessments will include complete motor and sensory examinations using the International Standards for Neurological Classification of Spinal Cord Injury (ISNCSCI). The AIS grade and ISNCSCI motor and sensory scores will be employed as exploratory assessments of the potential activity of PEG and magnesium, as well as to monitor outcome from the point of view of safety.

### Example 6

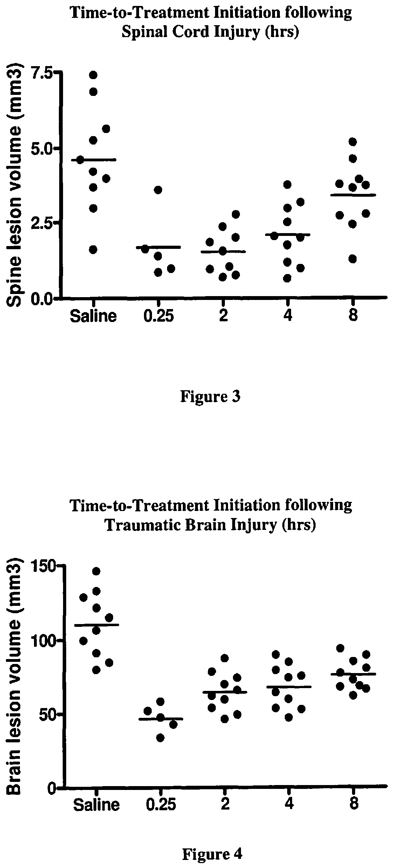

Figures 3 provides a graph showing time-to-treatment initiation following spinal cord injury and Figure 4 provides a graph showing time-to-treatment initiation following traumatic brain injury. Both figures reflect that the time following injury corresponds to the lesion volume in mm³.

Even though the disclosure herein has been described with reference to particular examples and embodiments, it is to be understood that these examples and embodiments are merely illustrative of the principles and applications of the present disclosure. It is therefore to be understood that numerous modifications may be made to the illustrative embodiments and that other arrangements may be devised without departing from the spirit and scope of the present disclosure as defined by the following claims.

## Claims

1. A composition for use in the treatment of a neuronal injury in a patient suffering therefrom, the composition comprising an injectable solution of magnesium, polyethylene glycol and an anti-oxidant, wherein the magnesium is magnesium chloride hexahydrate, the polyethylene glycol is polyethylene glycol 3350 (PEG3350) and the anti-oxidant comprises butylated hydroxytoluene (BHT), wherein the magnesium chloride hexahydrate and PEG3350 are packaged separately and then combined before administration, wherein the magnesium chloride hexahydrate is supplied in a first vial comprising an aqueous solution of magnesium where the magnesium is administered in a dosing range from 0.01 to 0.5 mmol of Mg ions/kg, and the PEG3350 together with the anti-oxidant comprising BHT is supplied in a second vial comprising an aqueous solution containing PEG3350 and sodium chloride where the PEG3350 is administered in a dosing range at 0.1 to 60% w/v, w/w and/or v/v, and the anti-oxidant comprising BHT is in an amount of 0.00001% to 5% w/v/, w/w and/or v/v of the composition, wherein at least three doses of the composition are administered to the patient.

2. The composition for use of claim 1, wherein the composition is mixed with saline, and at least six doses of the composition are administered to the patient.

3. The composition for use of claim 1, wherein the composition is administered by parenteral administration.

4. The composition for use of claim 1, wherein the composition is administered by local administration to a spinal cord.

5. The composition for use of claim 1, wherein the composition is administered by a bolus infusion, a continuous infusion, an intravenous administration, an intramuscular administration, an intrathecal administration, a subcutaneous administration, an epidural administration, an intra-articular administration, or any combinations thereof.

6. The composition for use of claim 2, wherein an initial dose is administered for 30 minutes within 12 hours post injury and the remaining doses are administered in 6 hour intervals.

7. The composition for use of claim 1, wherein a first dose of the composition is administered by or within 6 hours of the neuronal injury.

8. The composition for use of claim 1, wherein the composition is administered by or within 8, 10 or 12 hours of the neuronal injury.

9. The composition for use of claim 1, wherein the first vial is mixed with saline and the second vial is mixed with saline and administered to the patient.

10. The composition for use of claim 1, wherein the injectable solution comprises sodium chloride.

11. The composition for use of claim 1, wherein the neuronal injury comprises spinal cord injury, ischemic injury, hemorrhagic stroke injury, brain injury, traumatic brain injury, nerve injury and peripheral nerve injury.

12. The composition for use of claim 1, wherein the neuronal injury comprises spinal cord injury and traumatic brain injury.

## Patentansprüche

1. Zubereitung zur Verwendung bei der Behandlung einer neuronalen Verletzung in einem Patienten, der daran leidet, wobei die Zubereitung eine injizierbare Lösung von Magnesium, Polyethylenglykol und einem Oxidationsinhibitor umfasst, wobei das Magnesium Magnesiumchlorid-Hexahydrat ist, das Polyethylenglykol Polyethylenglykol 3350 (PEG3350) ist und der Oxidationsinhibitor butyliertes Hydroxytoluol (BHT) umfasst, wobei das Magnesiumchlorid-Hexahydrat und PEG3350 getrennt ver-packt sind und dann vor der Verabreichung zusammengegeben werden, wobei das Magnesiumchlorid-Hexahydrat in einem ersten Glasfläschchen bereitgestellt wird, das eine wäßrige Lösung von Magnesium umfasst, wo das Magnesium in einem Dosierungsbereich von 0,01 bis 0,5 mMol an Mg-Ionen/kg verabreicht wird, und das PEG3350 zusammen mit dem Oxidationsinhibitor, der BHT umfasst, in einem zweiten Glas-fläschchen bereitgestellt wird, das eine wäßrige Lösung um-fasst, die PEG3350 und Natriumchlorid enthält, wo das PEG3350 in einem Dosierungsbereich bei 0,1 bis 60 % w/v, w/w und/oder v/v verabreicht wird, und der BHT umfassende Oxidationsinhibitor in einer Menge von 0,00001 bis 5 % w/v, w/w und/oder v/v der Zubereitung vorliegt, wobei dem Patienten wenigstens drei Dosierungen der Zubereitung verabreicht werden.

2. Zubereitung zur Verwendung nach Anspruch 1, wobei die Zubereitung gemischt wird mit Salzlösung und wenigstens sechs Dosierungen der Zubereitung an den Patienten verabreicht werden.

3. Zubereitung zur Verwendung nach Anspruch 1, wobei die Zubereitung durch parenterale Verabreichung verabreicht wird.

4. Zubereitung zur Verwendung nach Anspruch 1, wobei die Zubereitung durch lokale Verabreichung an ein Rückenmark verabreicht wird.

5. Zubereitung zur Verwendung nach Anspruch 1, wobei die Zubereitung verabreicht wird durch eine Bolus-Infusion, eine kontinuierliche Infusion, eine intravenöse Verabreichung, eine intramuskuläre Verabreichung, eine intrathekale Verabreichung, eine subkutane Verabreichung, eine epidurale Verabreichung, eine intra-artikuläre Verabreichung oder irgendwelche Kombinationen davon.

6. Zubereitung zur Verwendung nach Anspruch 2, wobei eine Anfangsdosis während 30 Minuten innerhalb von 12 Stunden nach der Verletzung verabreicht wird und die verbleibenden Dosen in 6-Stunden-Intervallen verabreicht werden.

7. Zubereitung zur Verwendung nach Anspruch 1, wobei eine erste Dosis der Zubereitung verabreicht wird bei der neuronalen Verletzung oder innerhalb von 6 Stunden der neuronalen Verletzung.

8. Zubereitung zur Verwendung nach Anspruch 1, wobei die Zubereitung verabreicht wird bei der neuronalen Verletzung oder innerhalb von 8, 10 oder 12 Stunden der neuronalen Verletzung.

9. Zubereitung zur Verwendung nach Anspruch 1, wobei das erste Glasfläschchen mit Salzlösung gemischt wird und das zweite Glasfläschchen mit Salzlösung gemischt wird und an den Patienten verabreicht wird.

10. Zubereitung zur Verwendung nach Anspruch 1, wobei die injizierbare Lösung Natriumchlorid umfasst.

11. Zubereitung zur Verwendung nach Anspruch 1, wobei die neuronale Verletzung eine Rückenmarks-Verletzung, eine ischämische Verletzung, eine Verletzung in Form eines hämorrhagischen Schlaganfalls, eine Gehirn-Verletzung, eine traumatische Gehirn-Verletzung, eine Nerven-Verletzung und eine periphere Nerven-Verletzung umfasst.

12. Zubereitung zur Verwendung nach Anspruch 1, wobei die neuronale Verletzung eine Rückenmarks-Verletzung und eine traumatische Gehirn-Verletzung umfasst.

## Revendications

1. Composition pour une utilisation dans le traitement d'une lésion neuronale chez un patient en souffrant, la composition comprenant une solution injectable de magnésium, du polyéthylène glycol et un antioxydant, dans laquelle le magnésium est du chlorure de magnésium hexahydraté, le polyéthylène glycol est du polyéthylène glycol 3350 (PEG3350) et l'antioxydant comprend de l'hydroxytoluène butylé (BHT), dans laquelle le chlorure de magnésium hexahydraté et le PEG3350 sont conditionnés séparément et ensuite combinés avant l'administration, dans laquelle le chlorure de magnésium hexahydraté est fourni dans un premier flacon comprenant une solution aqueuse de magnésium où le magnésium est administré dans une plage de doses de 0,01 à 0,5 mmol d'ions Mg/kg, et le PEG3350 conjointement avec l'antioxydant comprenant du BHT est fourni dans un second flacon comprenant une solution aqueuse contenant du PEG3350 et du chlorure de sodium où le PEG3350 est administré dans une plage de doses de 0,1 à 60 % p/v, p/p et/ou v/v, et l'antioxydant comprenant du BHT est dans une quantité de 0,00001 % à 5 % p/v, p/p et/ou v/v de la composition, dans laquelle au moins trois doses de la composition sont administrées au patient.

2. Composition pour une utilisation selon la revendication 1, dans laquelle la composition est mélangée avec du sérum physiologique, et au moins six doses de la composition sont administrées au patient.

3. Composition pour une utilisation selon la revendication 1, dans laquelle la composition est administrée par administration parentérale.

4. Composition pour une utilisation selon la revendication 1, dans laquelle la composition est administrée par administration locale à une moelle épinière.

5. Composition pour une utilisation selon la revendication 1, dans laquelle la composition est administrée par une perfusion de bolus, une perfusion continue, une administration intraveineuse, une administration intramusculaire, une administration intrathécale, une administration sous-cutanée, une administration épidurale, une administration intra-articulaire, ou l'une quelconque de leurs combinaisons.

6. Composition pour une utilisation selon la revendication 2, dans laquelle une dose initiale est administrée pendant 30 minutes dans les 12 heures qui suivent la lésion et les doses restantes sont administrées à des intervalles de 6 heures.

7. Composition pour une utilisation selon la revendication 1, dans laquelle une première dose de la composition est administrée à ou dans les 6 heures qui suivent la lésion neuronale.

8. Composition pour une utilisation selon la revendication 1, dans laquelle la composition est administrée à ou dans les 8, 10 ou 12 heures qui suivent la lésion neuronale.

9. Composition pour une utilisation selon la revendication 1, dans laquelle le premier flacon est mélangé avec du sérum physiologique et le second flacon est mélangé avec du sérum physiologique et administré au patient.

10. Composition pour une utilisation selon la revendication 1, dans laquelle la solution injectable comprend du chlorure de sodium.

11. Composition pour une utilisation selon la revendication 1, dans laquelle la lésion neuronale comprend une lésion de la moelle épinière, une lésion ischémique, une lésion due à un accident vasculaire cérébral hémorragique, une lésion cérébrale, une lésion cérébrale traumatique, une lésion nerveuse et une lésion nerveuse périphérique.

12. Composition pour une utilisation selon la revendication 1, dans laquelle la lésion neuronale comprend une lésion de la moelle épinière et une lésion cérébrale traumatique.
